# NOUVEAU FASCICULE DE BREVET EUROPEEN

(11) **EP 0 991 426 B2**
(45) Date de publication et mention de la décision concernant l'opposition: **28.03.2007**
(45) Mention de la délivrance du brevet: 08.09.2004
(21) Numéro de dépôt: 98935067.3
(22) Date de dépôt: 30.06.1998
(51) Int. Cl.: A61K 48/00

(54) **PROCEDE DE TRANSFERT D'ACIDE NUCLEIQUE DANS LE MUSCLE STRIE**
VERABREICHUNG DER NUKLEINSÄURE IN DEN QUERGESTREIFTEN MUSKEL
METHOD FOR TRANSFERRING NUCLEIC ACID INTO THE STRIATED MUSCLE

(30) Priorité: 30.06.1997 FR 9708233; 01.12.1997 US 67488 P
(43) Date de publication de la demande: 12.04.2000
(73) Titulaire: Institut Gustave Roussy, 94805 Villejuif Cédex (FR); CENTRE NATIONAL DE LA RECHERCHE SCIENTIFIQUE (CNRS), 75016 Paris (FR)
(72) Inventeur: BUREAU, Michel, F-92210 Saint Cloud (FR); MIT, Lluis, F-91370 Verrières le Buisson (FR); SCHERMAN, Daniel, F-75012 Paris (FR)
(74) Mandataire: Warcoin, Jacques
(86) Numéro de dépôt international: PCT/FR1998/001400
(87) Numéro de publication internationale: WO 1999/001158

(56) Documents cités:
- WO-A-95/06129
- WO-A-96/14836
- WO-A-97/07826
- WO-A-98/43702
- HELLER ET AL: "IN VIVO GENE ELECTROINJECTION AND EXPRESSION IN RAT LIVER" FEBS LETTERS, vol. 389, 1996, pages 225-228, XP002058383 cité dans la demande
- NISHI ET AL: "HIGH-EFFICIENCY IN VIVO GENE TRANSFER USING INTRAARTERIAL PLASMID DNA INJECTION FOLLOWING IN VIVO ELECTROPORATION" CANCER RESEARCH, vol. 56, 1996, pages 1050-1055, XP002058384

## Description

La présente invention se rapporte à une amélioration très remarquable du transfert in vivo dans les cellules des muscles striés d'acides nucléiques ou d'acides nucléiques associés à des produits permettant d'augmenter le rendement de tels transferts, et à la combinaison d'un acide nucléique et du procédé de transfert selon l'invention pour leur utilisation pour la thérapie génique.

Le transfert de gènes dans une cellule donnée est à la base de la thérapie génique. Cependant, l'un des problèmes est de parvenir à faire pénétrer une quantité d'acide nucléique suffisante dans des cellules de l'hôte à traiter ; en effet, cet acide nucléique, en général un gène d'intérêt, doit être exprimé dans des cellules transfectées. L'une des approches retenue à cet égard a été l'intégration de l'acide nucléique dans des vecteurs viraux, en particulier dans des rétrovirus, des adénovirus ou des virus associés aux adénovirus. Ces systèmes mettent à profit les mécanismes de pénétration cellulaire développés par les virus, ainsi que leur protection contre la dégradation. Cependant, cette approche présente des inconvénients, et en particulier un risque de production de particules virales infectieuses susceptibles de dissémination dans l'organisme hôte, et, dans le cas des vecteurs rétroviraux, un risque de mutagénèse insertionnelle. De plus, la capacité d'insertion d'un gène thérapeutique ou vaccinal dans un génome viral demeure restreinte.

En tout état de cause, le développement de vecteurs viraux utilisables en thérapie génique impose d'avoir recours à des techniques complexes de virus défectifs et de lignées cellulaires de complémentation.

Une autre approche (Wolf et al. Science 247, 1465-68, 1990 ; Davis et al. Proc. Natl. Acad. Sci. USA 93, 7213-18, 1996) a donc consisté à administrer dans le muscle ou dans la circulation un acide nucléique de nature plasmidique, associé ou non à des composés destinés à favoriser sa transfection, comme des protéines, des liposomes, des lipides chargés ou des polymères cationiques tels que le polyéthylènimine, qui sont de bons agents de transfection in vitro (Behr et al. Proc. Natl. Acad. Sci. USA 86, 6982-6, 1989 ; Felgner et al. Proc. Natl. Acad. Sci. USA 84, 7413-7, 1987 ; Boussif et al. Proc. Natl. Acad. Sci. USA 92, 7297-301, 1995).

Depuis la publication initale de J.A. Wolff et al. montrant la capacité du tissu musculaire à incorporer de l'ADN injecté sous forme de plasmide libre (Wolff et al. Science 247, 1465-1468, 1990) de nombreux auteurs ont tenté d'améliorer ce processus (Manthorpe et al., 1993, Human Gene Ther. 4, 419-431 ; Wolff et al., 1991, BioTechniques 11, 474-485). Quelques tendances se dégagent de ces essais, comme notamment :
- l'utilisation de solutions mécaniques pour forcer l'entrée de l'ADN dans les cellules, en adsorbant l'ADN sur des billes propulsées ensuite sur les tissus (« gene gun ») (Sanders Williams et al., 1991, Proc. Natl. Acad. Sci. USA 88, 2726-2730 ; Fynan et al., 1993, BioTechniques 11, 474-485). Ces procédés se sont avérés efficaces dans des stratégies de vaccination, mais ne touchent que les couches superficielles des tissus. Dans le cas du muscle, leur utilisation nécessiterait un abord chirurgical pour permettre d'accéder au muscle, car les particules ne traversent pas les tissus cutanés ;
- l'injection d'ADN, non plus sous forme de plasmide libre, mais associé à des molécules susceptibles de servir de véhicule facilitant l'entrée des complexes dans les cellules. Les lipides cationiques, utilisés dans de nombreux autres procédés de transfection, se sont avérés jusqu'à l'heure actuelle décevants quant à une application dans le tissu musculaire, car ceux qui ont été testés se sont montrés inhibiteurs de la transfection (Schwartz et al., 1996, Gene Ther. 3, 405-411). Il en est de même pour les peptides et polymères cationiques (Manthorpe et aL, 1993, Human Gene Ther. 4, 419-431). Le seul cas d'association favorable semble être le mélange polyvinylalcool ou polyvinylpyrrolidone avec l'ADN. L'augmentation résultant de ces associations ne représente qu'un facteur inférieur à 10 par rapport à l'ADN injecté nu (Mumper et al., 1996, Pharmaceutical Research 13, 701-709) ;
- le prétraitement du muscle à injecter avec des solutions destinées à améliorer la diffusion et/ou la stabilité de l'ADN (Davis et al., 1993, Hum. Gene Ther. 4, 151-159), ou à favoriser l'entrée des acides nucléiques, par exemple l'induction de phénomènes de multiplication ou de régénération de cellules. Les traitements ont concerné en particulier l'utilisation d'anesthésiques locaux ou de cardiotoxine, de vasoconstricteurs, d'endotoxine ou d'autres molécules (Manthorpe et al., 1993, Human Gene Ther. 4, 419-431 ; Danko et al., 1994, Gene Ther. 1, 114-121 ; Vitadello et al., 1994, Hum. Gene Ther. 5, 11-18). Ces protocoles de prétraitement sont difficiles à gérer, la bupivacaïne en particulier nécessitant pour être efficace d'être injectée à des doses très proches des doses létales. La préinjection de sucrose hyperosmotique, destinée à améliorer la diffusion, n'augmente pas le niveau de la transfection dans le muscle (Davis et al., 1993).

L'électroporation, ou utilisation de champs électriques pour perméabiliser des cellules, est également utilisée in vitro pour favoriser la transfection d'ADN dans des cellules en culture. Toutefois, il était jusqu'à présent admis que ce phénomène répondait à un effet dépendant d'un seuil et que cette électroperméabilisation ne pouvait être observée que pour des champs électriques d'intensité relativement élevée, de l'ordre de 800 à 1 200 Volts/cm pour les cellules animales. Cette technique a également été proposée in vivo pour améliorer l'efficacité d'agents antitumoraux, comme la bléomycine, dans des tumeurs solides chez l'homme (brevet américain n° 5 468 228, L.M. Mir). Avec des impulsions de très courte durée (100 microsecondes), ces conditions électriques (800 à 1 200 Volts/cm) sont très bien adaptées au transfert intracellulaire de petites molécules. Ces conditions (impulsions de 100 microsecondes) ont été appliquées sans amélioration pour le transfert d'acides nucléiques in vivo dans le foie, où des champs inférieurs à 1 000 Volts/cm se sont révélés totalement inefficaces, et même inhibiteurs par rapport à l'injection d'ADN en l'absence d'impulsions électriques (brevet WO 97/07826 et Heller et al. FEBS Letters, 389, 225-8, 1996).

Cette technique présente d'ailleurs des difficultés d'application in vivo, car l'administration de champs d'une telle intensité peut provoquer des lésions tissulaires plus ou moins étendues, qui ne représentent pas un problème pour le traitement de tissus tumoraux mais qui peuvent représenter un inconvénient majeur pour le sujet sain ou pour le sujet malade lorsque l'acide nucléique est administré dans des tissus autres que les tissus tumoraux, en particulier dans le muscle strié.

La demande WO 98/43702 décrit une méthode d'introduction de composés pharmaceutiques et d'acides nucléiques dans le muscle squelettique en utilisant un courant constitué d'ondes bipolaires.

Alors que toutes les études citées mentionnent la nécessité de champs électriques élevés, de l'ordre de 1 000 Volts/cm, pour être efficace in vivo, de manière vraiment inattendue et remarquable, les demandeurs ont à présent montré que le transfert d'acides nucléiques dans des muscles in vivo pouvait être augmenté de façon très importante, sans effets indésirables, en soumettant le muscle à des impulsions électriques d'ondes unipolaires d'intensité faible, par exemple de 100 ou de 200 Volts/cm, et d'une durée relativement longue. De plus, les demandeurs ont constaté que la grande variabilité d'expression du transgène observée dans l'art antérieur de transfert d'ADN dans le muscle était notablement réduite par le procédé selon l'invention.

C'est pourquoi, la présente invention concerne un procédé de transfert d'acides nucléiques dans un ou plusieurs muscles striés in vivo, dans lequel les cellules des muscles sont mises en contact avec l'acide nucléique à transférer, par administration directe dans le tissu ou par administration topique ou systémique, et dans lequel le transfert est assuré par application auxdits muscles d'une ou de plusieurs impulsions électriques d'ondes unipolaires d'une intensité comprise entre 1 et 800 Volts/cm.

De préférence, l'intensité du champ est comprise entre 4 et 400 Volts/cm et la durée totale d'application est supérieure à 10 millisecondes. Le nombre d'impulsions utilisées est par exemple de 1 à 100 000 impulsions et la fréquence des impulsions est comprise entre 0,1 et 1000 Hertz. De préférence la fréquence des impulsions est comprise entre 0,2 et 100 Hertz. Les impulsions peuvent être aussi délivrées de manière irrégulière et la fonction qui décrit l'intensité du champ en fonction du temps peut être variable. A titre d'exemple, le champ électrique délivré peut résulter de la combinaison d'au moins un champ d'une intensité > à 400 V/cm et de préférence comprise entre 500 et 800 Volts/cm, de durée unitaire courte (< 1 msec), suivi de une ou plusieurs impulsions d'intensité plus faible , par exemple < 400 Volts/cm, et de préférence < 200 Volts /cm et de durée unitaire plus longue ( > 1 msec). L'intégrale de la fonction décrivant la variation du champ électrique avec le temps est supérieure à 1 kVₓmsec/cm. Selon un mode préféré de l'invention, cette intégrale est supérieure ou égale à 5 kVₓmsec/cm.

Selon un mode préféré de l'invention, l'intensité de champ des impulsions d'ondes unipolaires est comprise entre 30 et 300 Volts/cm.

Les impulsions électriques, par exemple à ondes carrées, sont des impulsions à ondes unipolaires oscillantes de durée limitée.

L'administration d'impulsions électriques peut être réalisée par toute méthode connue de l'homme du métier, par exemple :
- système d'électrodes externes placées de part et d'autre du tissu à traiter, notamment électrodes non invasives placées au contact de la peau,
- système d'électrodes implantées dans les tissus,
- système d'électrodes/injecteur permettant l'administration simultanée des acides nucléiques et du champ électrique.

Dans le cadre de la présente invention les termes transfert d'ADN ou d'acides nucléiques par application d'une ou plusieurs impulsions électriques, ainsi que les termes électrotransfert ou encore électrotransfection doivent être considérés comme équivalents et désignent le transfert d'acides nucléiques ou d'ADN par application ou en présence d'un champ électrique.

L'administration étant réalisée in vivo, il est parfois nécessaire d'avoir recours à des produits intermédiaires assurant la continuité électrique avec des électrodes externes non invasives. Il s'agira par exemple d'électrolyte sous forme de gel.

Les acides nucléiques peuvent être administrés par tout moyen approprié, mais sont de préférence injectés in vivo directement dans le muscle ou administrés par une autre voie, locale ou systémique, qui les rend disponibles à l'endroit d'application du champ électrique. Les acides nucléiques peuvent être administrés avec des agents permettant ou facilitant le transfert, comme cela a été mentionné précédemment. Notamment, ces acides nucléiques peuvent être libres en solution ou associés à des agents synthétiques, ou portés par des vecteurs viraux. Les agents synthétiques peuvent être des lipides ou des polymères connus de l'homme du métier, ou bien encore des éléments de ciblage permettant la fixation sur la membrane des tissus cibles. Parmi ces éléments, on peut citer des vecteurs portant des sucres, des peptides, des anticorps ou des récepteurs hormonaux.

On conçoit, dans ces conditions de l'invention, que l'administration des acides nucléiques puisse précéder, être simultanée ou même suivre l'application des champs électriques.

C'est pourquoi, la présente invention a également pour objet un acide nucléique et un champ électrique d'une intensité comprise entre 1 et 800 Volts/cm, comme produit de combinaison pour leur administration simultanée, séparée ou étalée dans le temps, au muscle strié in vivo. De préférence, l'intensité du champ est comprise entre 4 et 400 Volts/cm et, de manière encore plus préférée, l'intensité du champ est comprise entre 30 et 300 Volts/cm.

Le procédé selon la présente invention est utilisable dans la thérapie génique, c'est-à-dire la thérapie dans laquelle l'expression d'un gène transféré, mais également la modulation ou le blocage d'un gène, permet d'assurer le traitement d'une pathologie particulière.

De préférence, les cellules musculaires sont traitées dans le but d'une thérapie génique permettant :
- soit la correction des dysfonctionnements des cellules musculaires elles-mêmes (par exemple pour le traitement des myopathies liées à des déficiences génétiques),
- soit la sauvegarde et/ou la régénération de la vascularisation ou de l'innervation du muscle et d'autres muscles ou organes par des facteurs trophiques, neurotrophiques et angiogéniques produits par le transgène,
- soit la transformation du muscle en organe sécréteur de produits conduisant à un effet thérapeutique tels que le produit du gène lui-même (par exemple facteurs de régulation de thrombose et d'hémostase, facteurs trophiques, hormones comme l'insuline ou autres) ou tels qu'un métabolite actif synthétisé dans le muscle grâce à l'adjonction du gène thérapeutique,
- soit une application vaccinale ou immunostimulante.

Un autre objet de l'invention est l'association des impulsions électriques d'un champ à des compositions contenant les acides nucléiques formulées en vue de toute administration permettant d'accéder à un muscle strié par voie topique, cutanée, orale, vaginale, parentérale, intranasale, intraveineuse, intramusculaire, sous-cutanée, intra-oculaire, transdermique, etc. De préférence, les compositions pharmaceutiques de l'invention contiennent un véhicule pharmaceutiquement acceptable pour une formulation injectable, notamment pour une injection directe au niveau de l'organe désiré, ou pour toute autre administration. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations et le volume des injections peuvent être adaptées en fonction de différents paramètres, et notamment en fonction du mode d'administration utilisé, de la pathologie concernée, du gène à exprimer, ou encore de la durée du traitement recherchée.

Les acides nucléiques peuvent être d'origine synthétique ou biosynthétique, ou extraits de virus ou de cellules procaryotes ou de cellules eucaryotes provenant d'organismes unicellulaires (par exemple, levures) ou pluricellulaires. Ils peuvent être administrés en association de tout ou partie des composants de l'organisme d'origine et/ou du système de synthèse.

L'acide nucléique peut être un acide désoxyribonucléique ou un acide ribonucléique. Il peut s'agir de séquences d'origine naturelle ou artificielle, et notamment d'ADN génomique, d'ADNc, d'ARNm, d'ARNt et d'ARNr, de séquences hybrides ou de séquences synthétiques ou semisynthétiques d'oligonucléotides modifiés ou non. Ces acides nucléiques peuvent être obtenus par toute technique connue de l'homme du métier, et notamment par ciblage de banques, par synthèse chimique ou encore par des méthodes mixtes incluant la modification chimique ou enzymatique de séquences obtenues par ciblage de banques. Ils peuvent être modifiés chimiquement.

En particulier, l'acide nucléique peut être un ADN ou un ARN sens ou antisens ou à propriété catalytique comme un ribozyme. Par « antisens », on entend un acide nucléique ayant une séquence complémentaire à une séquence cible, par exemple une séquence d'ARNm dont on cherche à bloquer l'expression par hybridation sur la séquence cible. Par « sens », on entend un acide nucléique ayant une séquence homologue ou identique à une séquence cible, par exemple une séquence qui se lie à un facteur de transcription protéique et impliqué dans l'expression d'un gène donné. Selon un mode de réalisation préféré, l'acide nucléique comporte un gène d'intérêt et des éléments permettant l'expression dudit gène d'intérêt. Avantageusement, le fragment d'acide nucléique est sous forme d'un plasmide.

Les acides désoxyribonucléiques peuvent être simple ou double brin, de même que des oligonucléotides courts ou des séquences plus longues. Ils peuvent porter des gènes thérapeutiques, des séquences régulatrices de la transcription ou de la réplication, ou des régions de liaison à d'autres composants cellulaires, etc. Au sens de l'invention, on entend par « gène thérapeutique » notamment tout gène codant pour un ARN ou pour un produit protéique ayant un effet thérapeutique. Le produit protéique codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression du transgène permet par exemple de pallier à une expression insuffisante dans la cellule ou à l'expression d'une protéine inactive ou faiblement active en raison d'une modification, ou permet encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule (traitement des myopathies ou des déficits enzymatiques), ou permettre de lutter contre une pathologie, ou stimuler une réponse immunitaire.

Parmi les produits thérapeutiques au sens de la présente invention, on peut citer plus particulièrement les gènes codant pour
- les enzymes, comme l'α-1-antitrypsine, les proteinase (métalloproteinases, urokinase, uPA, tPA,...streptokinase), les protéases clivant des précurseurs pour libérer des produits actifs (ACE, ICE,...), ou leurs antagonistes (TIMP-1, tissue plasminogen activator inhibitor PAI, TFPI
- les dérivés sanguins comme les facteurs impliqués dans la coagulation : facteurs VII, VIII, IX, les facteurs du complement, la thrombine,
- les hormones, ou les enzymes impliquées dans la voie de synthèse des hormones, ou les facteurs impliqués dans le contrôle de la synthèse ou de l'excrétion ou de la sécrétion des hormones, telles que l'insuline, les facteurs proches de l'insuline (IGF), ou l'hormone de croissance, l'ACTH, les enzymes de synthèse des hormones sexuelles,
- les lymphokines et cytokines : interleukines, chemokines (CXC et CC), interférons, TNF, TGF, facteurs chimiotactiques ou activateurs comme MIF, MAF, PAF, MCP-1, l'eotaxine, LIF, etc. (brevet français n° 92 03120),
- les facteurs de croissance, par exemple les IGF, EGF, FGF, KGF, NGF, PDGF, PIGF, HGF, proliferin
- les facteurs angiogéniques tels que les VEGF ou FGF, angiopoietine 1 ou 2, l'endotheline
- les enzymes de synthèse de neurotransmetteurs,
- les facteurs trophiques, en particulier neurotrophiques pour le traitement des maladies neurodégénératives, des traumatismes ayant endommagé le système nerveux, ou des dégénerescences rétiniennes, tels que les membres de la famille des neurotrophines tels que le NGF, BDNF, NT3, NT4/5, NT6 leurs dérivés et gènes apparentés - les membres de la familles du CNTF tels que le CNTF, l'axokine, le LIF et leurs dérivés - l'IL6 et ses dérivés - la cardiotrophine et ses dérivés - le GDNF et ses dérivés - les membres de la famille des IGF, tels que l'IGF-1, l'IFGF-2 et leurs dérivés
- les membres de la famille de FGF, tels que le FGF 1, 2, 3, 4, 5, 6, 7, 8, 9 et leurs dérivés, le TGFβ
- les facteurs de croissance osseuse,
- les facteurs hématopoïétiques, comme erythropoietine, les GM-CSF, M-CSF, LIF, etc.,
- les protéines de l'architecture cellulaire comme la dystrophine ou une minidystrophine (brevet français n° 91 11947), , les gènes suicides (thymidine kinase, cytosine déaminase, enzymes à cytochrome P450), les gènes de l'hémoglobine ou d'autres transporteurs protéiques,
- les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple les lipases, la lipoprotéine lipase, la lipase hépatique, la lécithine cholestérol acyltransférase, la 7 alpha cholestérol hydroxylase, la phosphatidyl acide phosphatase, ou encore des protéines de transfert de lipides comme la protéine de transfert des esters de cholestérol et la protéine de transfert des phospholipides, une protéine de liaison des HDL ou encore un récepteur choisi par exemple parmi les récepteurs aux LDL, les récepteurs des chylomicrons-remnants et les récepteurs scavenger, etc. On peut, de plus, ajouter la leptine pour le traitement de l'obésité.
- les facteurs régulant la pression sanguine, comme les enzymes impliquées dans le métabolisme du NO, l'angiotensine, la bradykinine, vasopressine, l'ACE, la rénine, les enzymes codant pour les mécanismes de synthèse ou de relargage des prostaglandines, du thromboxane, ou de l'adenosine, les récepteurs de l'adénosine, les kallikreines et kallistatines, ANP, ANF, les facteurs diurétiques ou antidiurétiques, les facteurs impliqués dans la synthèse, le métabolisme ou le relargage des médiateurs comme l'histamine, la sérotonine, les cathécholamines, les neuropeptides,
- les facteurs anti-angiogéniques comme le ligand de Tie-1 et de Tie-2, l'angiostatine, le facteur ATF, les dérivés du plasminogène, l'endothéline, les thrombospondines 1 et 2, le PF-4, l'interféron α ou β, l'interleukine 12, le TNFα, le récepteur de l'urokinase, flt1, KDR, PAI1, PAI2, TIMP1, le fragment prolactine
- les facteurs protégeant contre l'apoptose, comme la famille AKT,
- les protéines susceptible d'induire une mort cellulaire, soit actives par elles-mêmes comme les caspases, soit de type "pro-drogues" nécéssitant une activation par d'autres facteurs, soit les protéines activant des pro-drogues en agent provoquant une mort cellulaire, comme la thymidine kinase du virus herpétique, les désaminase, permettant en particulier d'envisager des thérapies anti-cancéreures,
- les protéines impliquées dans les contacts et l'adhésion inter-cellulaires : VCAM, PECAM, ELAM, ICAM, intégrines, cathenines,
- les protéines de la matrice extra-cellulaire,
- les protéines impliquées dans la migration des cellules
- les protéines de type transduction du signal, type FAK, MEKK, p38 kinase, tyrosines kinases, serines- threonines kinases,
- les protéines impliquées dans la régulation du cycle cellulaire (p21, p16, cyclines, ...) ainsi que les protéines mutantes ou dérivées dominant négatif bloquant le cycle cellulaire et pouvant le cas échéant induire l'apoptose.
- les facteurs de transcription : jun, fos, AP1, p53,...et lesprotéines dela cascade de signalisation de p53.
- les protéines de structure de la cellule, comme les filaments intermédiaires (vimentine, desmine, keratines), la dystrophine, les protéines impliquées dans la contractilité et le contrôle de la contractibilité musculaire, en particulier les protéines impliquées dans le métabolisme calcique et les flux de calcium dans les cellules (SERCA, ...).
Dans les cas de protéines fonctionnant par des systèmes ligand et récepteurs, il est envisagable d'utiliser le ligand ou le récepteur (ex. FGF-R, VEGF-R, ...). On peut également citer des gènes codant pour des fragments ou des mutants de protéines de ligands ou de récepteurs, notamment des protéines précitées, présentant soit une activité supérieure à la protéine entière, soit une activité antagoniste, voire même de type " dominant négatif" par rapport à la protéine initiale (par exemple fragments de récepteurs inhibant la disponibilité de protéines circulantes, associés ou non avec des séquences induisant une sécrétion de ces fragments par rapport à un ancrage dans la membrane cellulaire, ou d'autres systèmes de modification du trafic intracellulaire de ces systèmes ligand- récepteurs de façon à détourner la disponibilité d'un des éléments) soit même possédant une activité propre distincte de celle de la protéine totale (ex. ATF).

Parmi les autres protéines ou peptides pouvant être sécrétés par le musclé, il est important de souligner les anticorps, les fragments variables d'anticorps simple chaîne (ScFv) ou tout autre fragment d'anticorps possédant des capacités de reconnaissance pour son utilisation en immunothérapie, par exemple pour le traitement des maladies infectieuses, des tumeurs, des maladies auto-immunes telles que la sclérose en plaques (anticorps antiidiotype), ainsi que les ScFv se fixant sur les cytokines pro-inflammatoires telles que par exemple IL1 et TNFα pour le traitement de l'arthrite rhumatoïde. D'autres protéines d'intérêt sont, de façon non limitative, des récepteurs solubles, comme par exemple le récepteur CD4 soluble ou le récepteur soluble du TNF pour la thérapie anti-HIV, le récepteur TNFα ou le récepteur soluble IL1 pour le traitement de l'arthrite rhumatoïde, le récepteur soluble de l'acétylcholine pour le traitement de la myasthénie ; des peptides substrats ou inhibiteurs d'enzymes, ou bien des peptides agonistes ou antagonistes de récepteurs ou de protéines d'adhésion comme par exemple pour le traitement de l'asthme, de la thrombose de la resténose, des métastases ou de l'inflammation ; des protéines artificielles, chimériques ou tronquées. Parmi les hormones d'intérêt essentiel, on peut citer l'insuline dans le cas du diabète, l'hormone de croissance et la calcitonine. On peut citer encore des protéines capables d'induire une immunité antitumorale ou stimuler la réponse immunitaire (IL2, GM-CSF, IL12, etc.). Enfin on peut citer les cytokines qui diminuent la réponse T_{H1} telles que IL10, IL4 et IL13.

Les nombreux exemples qui précèdent et ceux qui suivent illustrent l'étendue potentielle du champ d'application de la présente invention.

L'acide nucléique thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaire d'ARNm cellulaires et bloquer ainsi leur traduction en protéine, selon la technique décrite dans le brevet européen n° 140 308. Les gènes thérapeutiques comprennent également les séquences codant pour des ribozymes, qui sont capables de détruire sélectivement des ARN cibles (brevet européen n° 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins, soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (brevet européen n° 185 573), du virus de la pseudo-rage, du « syncitia forming virus », d'autres virus ou encore d'antigènes spécifiques de tumeurs comme les protéines MAGE (brevet européen n° 259 212), telles que les protéines MAGE 1, MAGE 2, ou des antigènes pouvant stimuler une réponse anti-tumorale telles que des protéines heat shock bactériennes.

Préférentiellement, l'acide nucléique comprend également des séquences permettant et/ou favorisant l'expression dans le muscle du gène thérapeutique et/ou du gène codant pour le peptide antigénique. Il peut s'agir des séquences qui sont naturellement responsables de l'expression du gène considéré lorsque ces séquences sont susceptibles de fonctionner dans la cellule transfectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire transfecter. Parmi les promoteurs eucaryotes, on peut utiliser tout promoteur ou séquence dérivée stimulant ou réprimant la transcription d'un gène de façon spécifique ou non, forte ou faible. Il peut s'agir en particulier de promoteurs ubiquitaires (HPRT, vimentine, α-actine, tubuline, etc.) , de promoteurs de gènes thérapeutiques (type MDR, CFTR, etc.), de promoteurs spécifiques de tissus (type promoteurs des gènes de la desmine, des myosines, de créatine kinase, de phophoglycérate kinase) ou encore de promoteurs répondant à un stimulus tels que des promoteurs répondant aux hormones naturelles (récepteur des hormones stéroïdes, récepteur de l'acide rétinoïque, etc.) ou un promoteur régulé par les antibiotiques (tétracycline, rapamycine, etc ), de promoteurs répondant à un régime alimentaire comme les promoteurs répondant aux fibrates, ou d'autres promoteurs répondant à d'autres molécules d'origine naturelle ou synthétique. De même, il peut s'agir de séquences promotrices issues du génome d'un virus. A cet égard, on peut citer par exemple les promoteurs des gènes EIA de l'adénovirus, MLP, ou de promoteurs issus des génomes des virus CMV, RSV, SV40, etc. Il peut s'agir de promoteurs inductibles ou répressibles. En outre, ces séquences d'expression peuvent être modifiées par addition de séquences d'activation, de régulation, permettant une expression conditionnelle, transitoire, une expression tissu-spécifique ou majoritaire, etc.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule, comme par exemple les peroxisomes, les lysosomes, et les mitochondries pour le traitement par exemple des maladies génétiques mitochondriales.

D'autres gènes présentant un intérêt ont été notamment décrits par McKusick, V.A. Mendelian (Inheritance in man, catalogs of autosomal dominant, autosomal recessive, and X-linked phenotypes. Eighth edition. John Hopkins University Press (1988)), et dans Stanbury, J.B. et al. (The metabolic basis of inherited disease, Fith edition. McGraw-Hill (1983)). Les gènes d'intérêt recouvrent les protéines impliquées dans le métabolisme des acides aminés, des lipides et des autres constituants de la cellule.

On peut ainsi citer de manière non limitative les gènes associés aux maladies du métabolisme des carbohydrates comme par exemple fructose-1-phosphate aldolase, fructose-1,6-diphosphatase, glucose-6-phosphatase, α-1,4-glucosidase lysosomale, amylo-1,6-glucosidase, amylo-(1,4:1,6)-transglucosidase, phosphorylase musculaire, phosphofructokinase musculaire, phosphorylase-b-kinase, galactose-1-phosphate uridyl transférase, toutes les enzymes du complexe pyruvate déshydrogénase, pyruvate carboxylase, 2-oxoglutarate glyoxylase carboxylase, D-glycérate déhydrogénase.

On peut également citer :
- les gènes associés avec des maladies du métabolisme des amino-acides comme par exemple phénylalanine hydroxylase, dihydrobioptérine synthétase, tyrosine aminotransférase, tyrosinase, histidinase, fumarylacéto-acétase, glutathion synthétase, γ-glutamylcystéine synthétase, ornithine-δ-aminotransférase, carbamoylphosphate synthétase, ornithine carbamoyltransférase, argininosuccinate synthétase, argininosuccinate lyase, arginase, L-lysine déhydrogénase, L-lysine kétoglutarate réductase, valine transaminase, leucine isoleucine transaminase, décarboxylase des 2-céto-acides à chaîne ramifiée, isovaléryl-CoA déhydrogénase, acyl-CoA déhydrogénase, 3-hydroxy-3-méthylglutaryl-CoA lyase, acétoacétyl-CoA 3-kétothiolase, propionyl-CoA carboxylase, méthylmalonyl-CoA mutase, ATP :cobalamine adénosyltransférase, dihydrofolate réductase, méthylène tétrahydrofolate réductase, cystathionine β-synthétase, le complexe sarcosine déshydrogénase, les protéines appartenant au système de clivage de la glycine, β-alanine transaminase, carnosinase sérique, homocarnosinase cérébrale.
- Les gènes associés avec des maladies du métabolisme des graisses et des acides gras, comme par exemple lipoprotéine lipase, apolipoprotéine C-II, apolipoprotéine E, d'autres apolipoprotéines, lécithine cholestérolacyltransférase, récepteur des LDL, stérol hydroxylase du foie, « acide phytanique » α-hydroxylase.
- Les gènes associés avec des déficiences lysosomales, comme par exemple α-L-iduronidase lysosomale, iduronate sulfatase lysosomale, héparan N-sulfatase lysosomale, N-acétayl-α-D-glucosaminidase lysosomale, acétyl-CoA : α-glucosamine N-acétyltransférase lysosomale, N-acétyl-α-D-glucosamine 6-sulfatase lysosomale, galactosamine 6-sulfate sulfatase lysosomale, β-galactosidase lysosomale, arylsulfatase B lysosomale, β-glucuronidase lysosomale, N-acétylglucosaminyl-phosphotransférase, α-D-mannosidase lysosomale, α-neuraminidase lysosomale, aspartylglycosaminidase lysosomale, α-L-fucosidase lysosomale, lipase acide lysosomale, céramidase acide lysosomale, sphingomyelinase lysosomale, glucocérébrosidase lysosomale et galactocérébrosidase lysosomale, galactosylcéramidase lysosomale, arylsulfatase A lysosomale, α-galactosidase A, β-galactosidase acide lysosomale, chaîne α de l'hexosaminidase A lysosomale.

On peut également citer, de façon non restrictive, les gènes associés avec des maladies du métabolisme des stéroïdes et des lipides, les gènes associés avec des maladies du métabolisme des purines et des pyrimidines, les gènes associés à des maladies du métabolisme de la porphyrine et de l'hème, les gènes associés à des maladies du métabolisme du tissu conjonctif, des s et des os ainsi que les gènes associés avec des maladies du sang et des organes hématopoïétiques, des muscles (myopathie), du système nerveux (maladies neurodégénératives) ou de l'appareil circulatoire (traitement des ischémies et de la sténose par exemple) et les gènes impliqués dans les maladies génétiques mitochondriales.

Dans le procédé suivant l'invention, l'acide nucléique peut être associé à tout type de vecteurs ou toute combinaison de ces vecteurs permettant d'améliorer le transfert de gènes, par exemple, de façon non limitative, à des vecteurs tels que des virus, des agents synthétiques ou biosynthétiques (par exemple lipidiques, polypeptidiques, glycosidiques ou polymériques), ou encore des billes propulsées ou non. Les acides nucléiques peuvent aussi être injectés dans un muscle qui a été soumis à un traitement visant à améliorer le transfert de gènes, par exemple un traitement de nature pharmacologique en application locale ou systémique, ou un traitement enzymatique, perméabilisant (utilisation de tensioactifs), chirurgical, mécanique, thermique ou physique.

L'avantage de l'utilisation du muscle en thérapie génique réside dans de nombreux facteurs :
- la stabilité remarquable de l'expression des transgènes, supérieure à plusieurs mois, et permettant donc la production stable et soutenue d'une protéine thérapeutique intramusculaire ou sécrétée,
- la facilité d'accès au tissu musculaire, permettant une administration directe, rapide et non dangereuse dans un organe non vital,
- le volume important de la masse musculaire, permettant de multiples sites d'administration,
- la capacité sécrétrice amplement démontrée du muscle.

A ces avantages, s'ajoute la sécurité apportée par le traitement local lié à l'utilisation de champs électriques locaux et ciblés.

De par l'ensemble de ces avantages et la sécurité liée à l'utilisation de champs faibles, la présente invention pourrait s'appliquer au niveau du muscle cardiaque pour le traitement de cardiopathies, par exemple en utilisant un défibrilateur adapté. Elle pourrait s'appliquer aussi au traitement de la resténose par l'expression de gènes inhibiteurs de la prolifération des cellules musculaires lisses comme la protéine GAX.

La combinaison de champs peu intenses et de durées d'administration longues appliquée notamment aux muscles in vivo améliore la transfection des acides nucléiques sans amener de détériorations notables des tissus. Ces résultats améliorent le rendement des transferts d'ADN dans le cadre de la thérapie génique mettant en oeuvre les acides nucléiques.

En conséquence, les avantages du tissu musculaire associés au procédé selon l'invention permettent, pour la première fois, d'envisager de produire par thérapie génique un agent à des doses physiologiques et/ou thérapeutiques, soit dans les cellules musculaires, soit sécrété dans leur voisinage ou dans la circulation sanguine ou lymphatique. De plus, le procédé selon l'invention permet, pour la première fois, la modulation fine et le contrôle de la quantité efficace de transgène exprimé par la possibilité de moduler le volume du tissu musculaire à transfecter, par exemple avec des sites multiples d'administration, ou encore la possibilité de moduler le nombre, la forme, la surface et la disposition des électrodes. Un élément de contrôle supplémentaire provient de la possibilité de moduler l'efficacité de la transfection par la variation de l'intensité de champ, du nombre de la durée et de la fréquence des impulsions, et évidemment suivant l'état de l'art, la quantité et le volume d'administration des acides nucléiques. On peut ainsi obtenir un niveau de transfection approprié au niveau de production ou de sécrétion désiré. Le procédé permet enfin un surcroît de sécurité par rapport aux méthodes chimiques ou virales de transfert de gènes in vivo, pour lesquelles l'atteinte d'organes autres que l'organe cible ne peut pas être totalement exclue et maîtrisée. En effet, le procédé selon l'invention permet le contrôle de la localisation des tissus transfectés (strictement liée au volume de tissu soumis aux impulsions électriques locales) et apporte donc la possibilité d'un retour à la situation initale par l'ablation totale ou partielle du muscle, rendue possible par le caractère non vital de ce tissu et par ses capacités de régénération. Cette grande souplesse d'utilisation permet d'optimiser le procédé suivant l'espèce animale (applications humaines et vétérinaires), l'âge du sujet, son état physiologique et/ou pathologique.

Le procédé selon l'invention permet, en outre, pour la première fois, de transfecter des acides nucléiques de grande taille contrairement aux méthodes virales qui sont limitées par la taille de la capside. Cette possibilité est essentielle pour le transfert de gènes de très grande taille comme celui de la dystrophine ou de gènes avec des introns et/ou des éléments régulateurs de grande taille, ce qui est nécessaire par exemple pour une production physiologiquement régulée d'hormones. Cette possibilité est essentielle pour le transfert d'épisomes ou de chromosomes artificiels de levure ou de minichromosomes.

Les exemples qui suivent sont destinés à illustrer de manière non limitative l'invention.

Dans ces exemples, on se réfèrera aux figures suivantes :
- Figure 1 : Effets d'impulsions électriques d'intensité de champ élevé sur la transfection d'ADN plasmidique pXL2774 dans le muscle tibial cranial chez la souris; valeurs moyennes ± SEM,
- Figure 2 : Effets d'impulsions électriques d'intensité de champ intermédiaire sur la transfection d'ADN plasmidique pXL2774 dans le muscle tibial cranial chez la souris; valeurs moyennes ± SEM,
- Figure 3 : Effets d'impulsions électriques d'intensité de champ faible et de différentes durées sur la transfection d'ADN plasmidique pXL2774. dans le muscle tibial cranial chez la souris; valeurs moyennes ± SEM,
- Figure 4 : Effets d'impulsions électriques d'intensité de champ faible et de différentes durées sur la transfection d'ADN plasmidique pXL2774 dans le muscle tibial cranial chez la souris; valeurs moyennes ± SEM,
- Figure 5 : Efficacité de l'électrotransfection de l'ADN plasmidique pXL2774 dans le muscle tibial cranial de la souris aux intensités de champs électriques faibles : valeurs moyennes ± SEM.
- Figure 6 :Cinétique d'expression de la luciférase dans le muscle tibial cranial de souris. Administration du plasmide pXL2774 avec électrotransfert (■ ) et sans électrotransfert ( X) ; valeurs moyennes ± SEM.
- Figure 7 : Niveau d'expression du transgène en fonction la dose d'ADN administrée, avec électrotransfert (● ) et sans électrotransfert ( □).
- Figure 8 : Effet de différents type d'électrodes sur l'efficacité de l'électrotransfert.
- Figure 9 : Cinétique de la concentration sérique en phophatase alcaline sécrétée. Administration du plasmide pXL3010 avec électrotransfert (■ ) et sans électrotransfert ( ◆) ; valeurs moyennes ± SEM.
- Figure 10 : Cinétique d'expression du FGF1 dans le muscle avec électrotransfert (barres d'histogramme blanches), et sans électrotransfert (barres d'histogramme noires).
- Figure 11 : cartes des plasmides pXL3179 et pXL3212.
- Figure 12 : cartes des plasmides pXL3388 et pXL3031.
- Figure 13 : cartes des plasmides pXL3004 et pXL3010.
- Figure 14 : cartes des plasmides pXL3149 et pXL3096.
- Figure 15 : cartes des plasmides pXL3353 et pXL3354.
- Figure 16 : carte du plasmide pXL3348

### Exemple 1 : expérience effectuée dans les conditions de l'état de la technique antérieure dans laquelle les champs électriques se montrent inhibiteurs de la transfection

Les conditions standards d'électroporation, telles que celles utilisées dans l'art antérieur et qui ont été discutées ci-avant, ont été testées et se sont avérées être inefficaces, voire même avoir une action inhibitrice sur le transert d'acides nucléiques (ADN plasmidique) dans le muscle strié.

### Matériel et Méthodes - Conditions opératoires générales

Dans cet exemple, les produits suivants ont été utilisés :

ADN pXL2774 (brevet PCT/FR 96/01414) est un ADN plasmidique comportant le gène rapporteur de la luciférase. Les autres produits sont disponibles auprès de fournisseurs du commerce : Kétamine, Xylazine, Sérum physiologique (NaCl 0,9 %).

Un oscilloscope et un générateur d'impulsions électriques (rectangulaires ou carrées) du commerce (Electro-pulsateur PS 15, Jouan, France) ont été utilisés. Les électrodes utilisées sont des électrodes plates en acier inoxydable distantes de 1 à 15 mm.

L'expérience est réalisée chez la souris C57 B1/6. Les souris provenant de différentes cages sont réparties au hasard avant l'expérience ("randomisation").

Les souris sont anesthésiées par un mélange kétamine, xylazine. La solution de plasmide (30 µl d'une solution à 500 µg/ml de NaCl 0,9%) est injectée longitudinalement à travers la peau dans le muscle tibial cranial des pattes gauche et droite à l'aide d'une seringue hamilton. Les deux électrodes sont enduites d'un gel conducteur et la patte injectée est placée entre les électrodes au contact de celles-ci.

Les impulsions électriques sont appliquées perpendiculairement à l'axe du muscle à l'aide d'un générateur d'impulsions carrées, une minute après l'injection. Un oscilloscope permet de contrôler l'intensité en Volts (les valeurs indiquées dans les exemples représentent les valeurs maximales), la durée en millisecondes et la fréquence en hertz des impulsions délivrées, qui est de 1 Hz. 8 impulsions consécutives sont délivrées.

Pour l'évaluation de la transfection du muscle, les souris sont euthanasiées 7 jours après l'administration du plasmide. Les muscles tibial cranial des pattes gauche et droite sont alors prélevés, pesés, mis dans du tampon de lyse et broyés. La suspension obtenue est centrifugée afin d'obtenir un surnageant clair. La mesure de l'activité luciférase est réalisée sur 10 µl de surnageant à l'aide d'un luminomètre du commerce dans lequel le substrat est ajouté automatiquement à la solution. L'intensité de la réaction lumineuse est donnée en RLU (Relative Luminescence Unit) pour un muscle connaissant le volume total de suspension (1.10⁶ RLU sont équivalents à 30 pg de luciférase). Chaque condition expérimentale est testée sur 10 points : 5 animaux injectés en bilatéral. Les comparaisons statistiques sont réalisées à l'aide de tests non paramétriques.

### Résultats et discussion.

Deux figures, dont l'échelle est linéaire ou logarithmique, illustrent les résultats.

Dans cette première expérience on a testé les effets d'un champ électrique de 800 à 1200 Volts/cm qui permet l'électroporation de tumeurs (Mir et al. Eur. J. Cancer 27, 68, 1991).

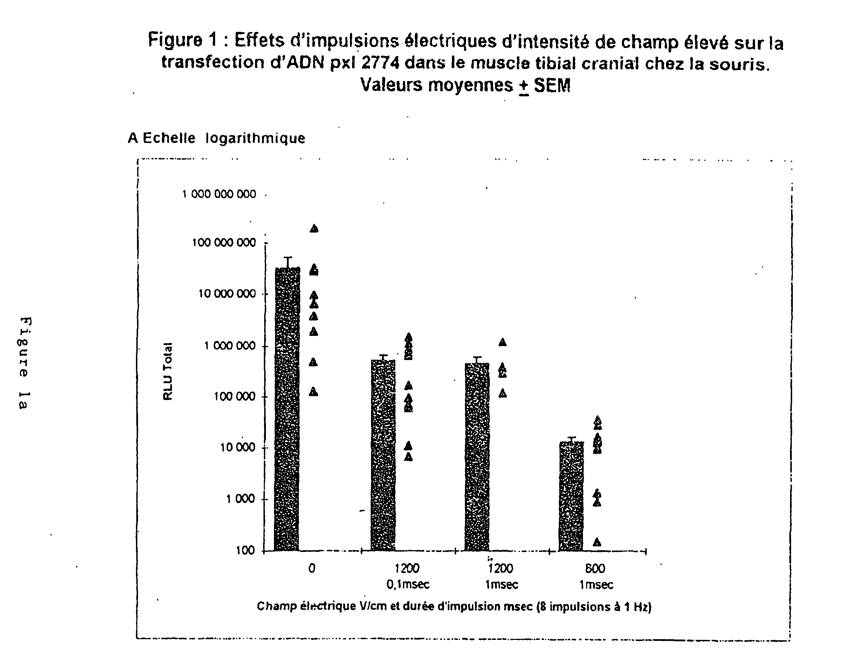

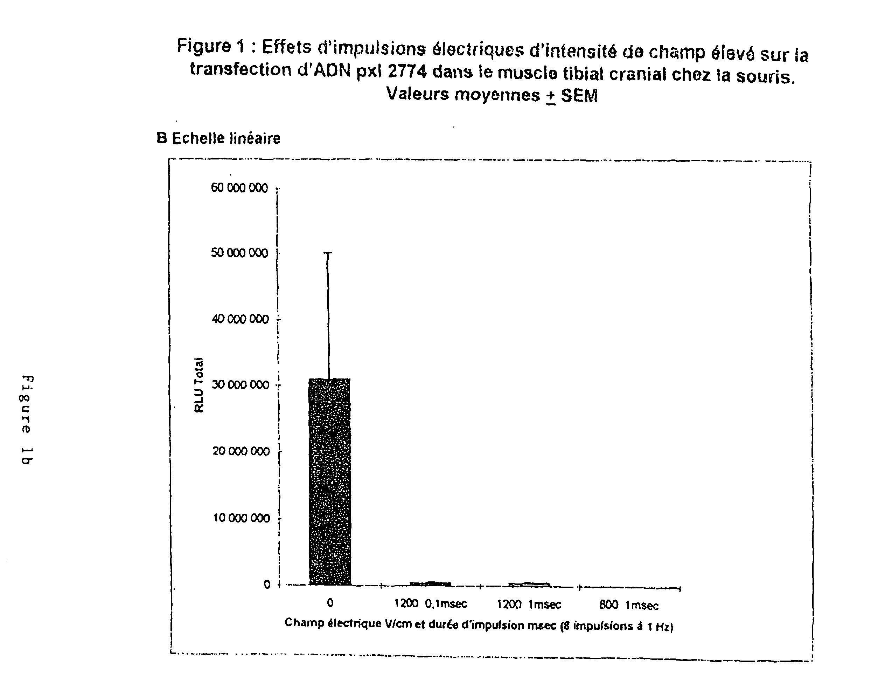

On constate, d'après la figure 1, que, relativement au groupe contrôle, où l'ADN est injecté sans impulsion électrique :
- avec 8 impulsions de 1200 Volts/cm et d'une durée de 0,1 msec, la valeur moyenne de l'activité luciférase est beaucoup plus faible,
- avec des impulsions de 1200 Volts/cm et de 1 msec, 3 animaux sont morts, la valeur moyenne de l'activité luciférase est beaucoup plus faible,
- avec des impulsions de 800 Volts/cm et de 1 msec la valeur moyenne de l'activité luciférase est aussi significativement réduite.

La plupart des muscles ayant subi l'action du champ électrique sont visiblement altérés (friables et d'aspect blanchâtre).

### Exemple 2 : expérience d'électrotransfert d'acides nucléiques à des champs électriques modérés

Cette expérience est réalisée avec des souris C57 B1/6. Mis à part l'intensité de champ électrique des impulsions et leur durée, les conditions de réalisation sont celles de l'exemple 1.

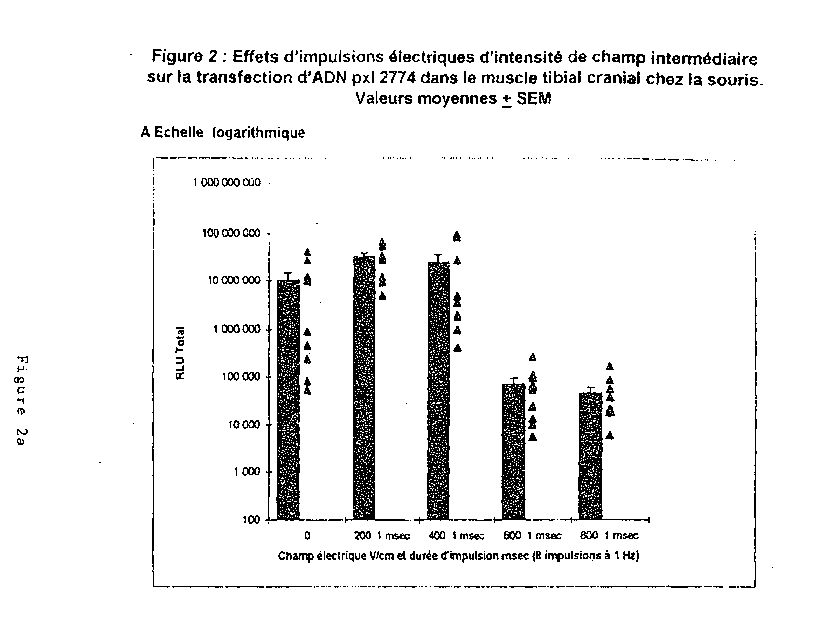

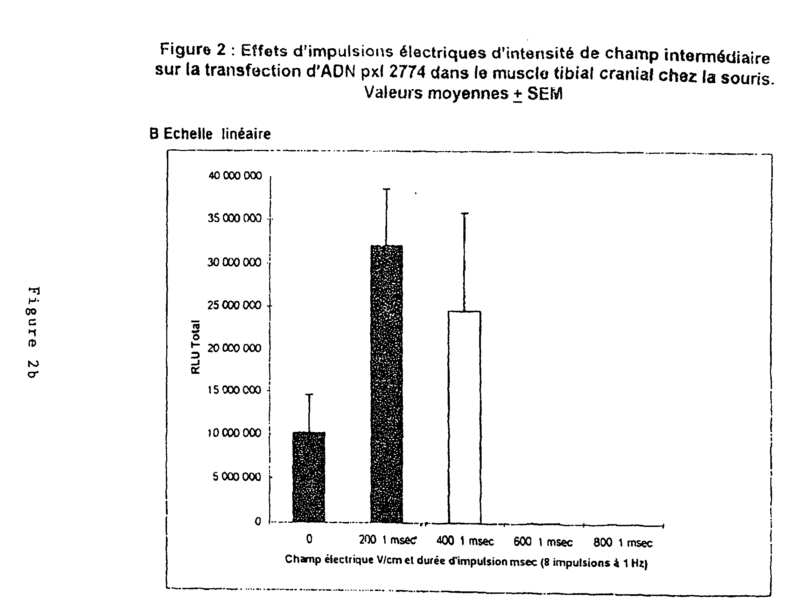

Les résultats sont montrés à la figure 2. On reproduit le résultat de l'exemple 1, c'est-à-dire l'effet inhibiteur d'une série de 8 impulsions à 800 Volts/cm d'une durée de 1 msec sur l'activité luciférase détectée dans le muscle. Avec un champ de 600 Volts/cm, on observe la même inhibition et la même altération du tissu musculaire. Par contre, de façon remarquable et surprenante, la diminution du voltage permet de ne plus altérer visiblement les muscles, et, de plus, à 400 et 200 Volts/cm le niveau de transfection des muscles est en moyenne supérieur à celui obtenu sur les muscles non soumis à un champ. Il est à noter que, relativement au groupe témoin (non soumis à un champ électrique), la dispersion des valeurs de l'activité luciférase est diminuée à 200 Volts/cm (SEM = 20,59% de la valeur moyenne contre 43,32% en l'absence de champ électrique (figure 2A)).

### Exemple 3 : expérience d'électrotransfert d'acides nucléiques avec des impulsions de faible intensité de champ montrant une très forte stimulation de l'expression du transgène

Cette expérience est réalisée avec des souris C57 B1/6. Mis à part l'intensité de champ électrique des impulsions et leur durée, et le fait que les impulsions sont délivrées 25 secondes après l'injection de l'ADN, les conditions de réalisation sont celles des exemples précédents.

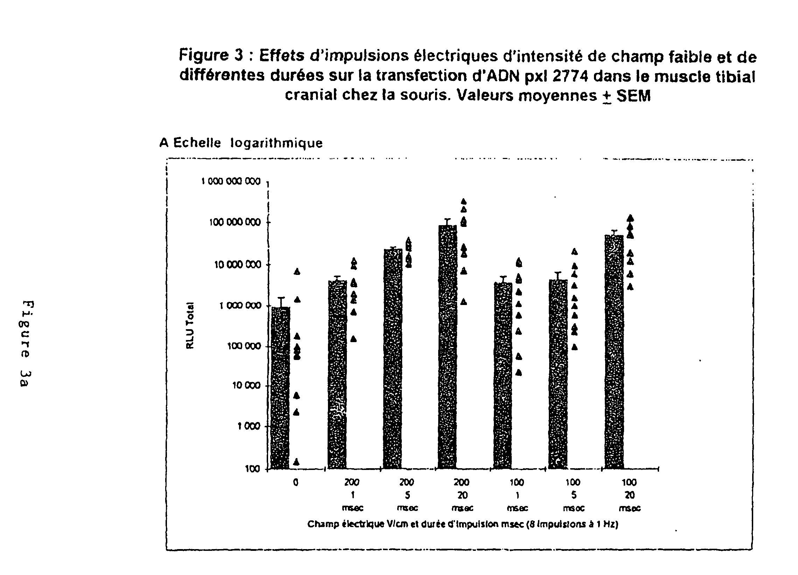

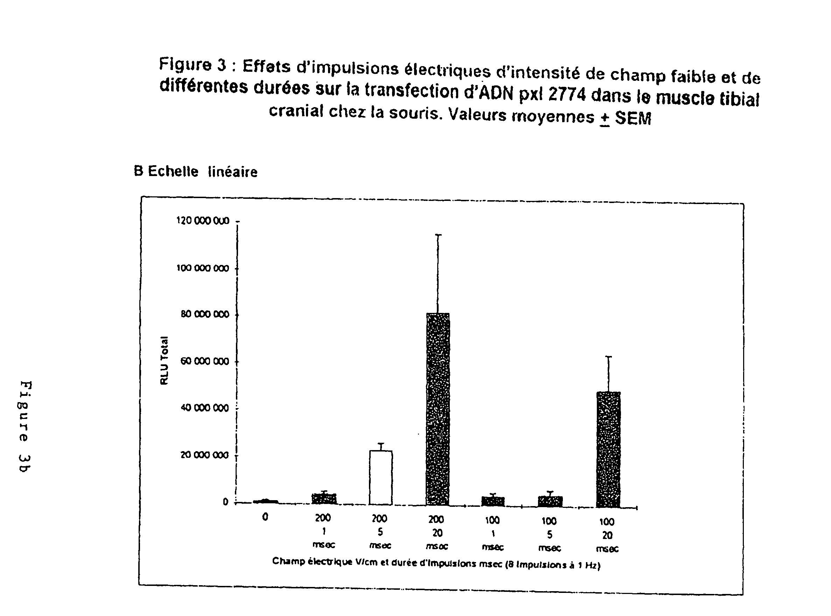

Les résultats sont montrés à la figure 3. La valeur moyenne de l'expression du transgène luciférase est nettement augmentée avec une durée d'impulsion de 20 msec à 100 Volts/cm, et à partir d'une durée d'impulsion de 5 msec à 200 Volts/cm.

Cette expérience montre aussi clairement que la valeur moyenne de l'activité luciférase obtenue par électrotransfection de l'ADN dans le muscle est une fonction de la durée des impulsions électriques, lorsqu'on emploie des voltages de 200 et 100 Volts/cm. On note aussi que la dispersion des valeurs est notablement réduite pour les groupes de muscles électrotransfectés (figure 3A). En l'absence d'impulsions électriques (contrôle), la SEM représente 77,43% de la valeur moyenne alors que la SEM relative de la moyenne est réduite à 14% (200 Volts/cm, 5 msec), 41,27% (200 Volts/cm, 20 msec) et entre 30% et 48% pour l'électrotransfert à 100 Volts/cm de champ électrique.

Dans la meilleure condition de cette expérience, on améliore par un facteur de 89,7 l'expression du transgène par rapport au contrôle injecté en l'absence d'impulsions électriques.

### Exemple 4 : expérience d'électrotransfert d'acides nucléiques dans le muscle à 200 Volts/cm montrant une augmentation de l'expression du transgène d'un facteur supérieur à 200

Cette expérience est effectuée chez les souris DBA 2, avec des impulsions électriques d'une intensité de champ de 200 Volts/cm et de durée variable, les autres conditions de cette expérience étant celles de l'exemple 3.

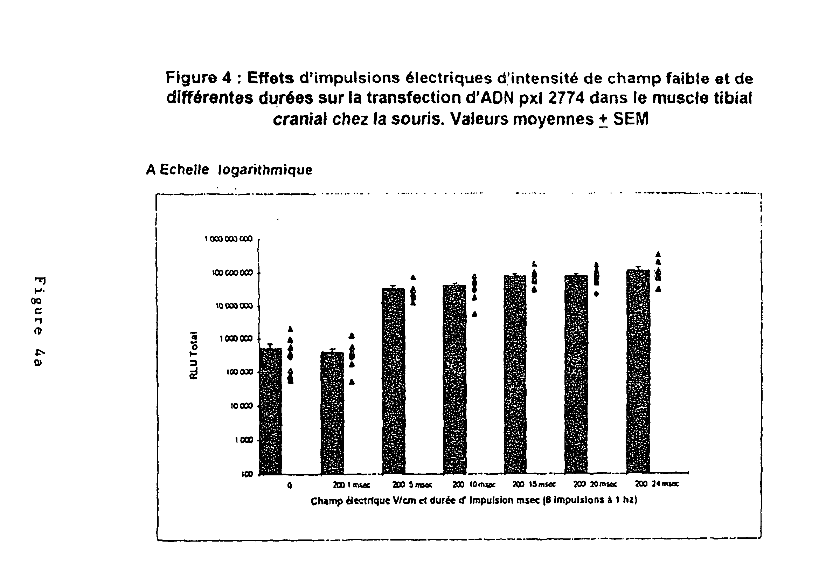

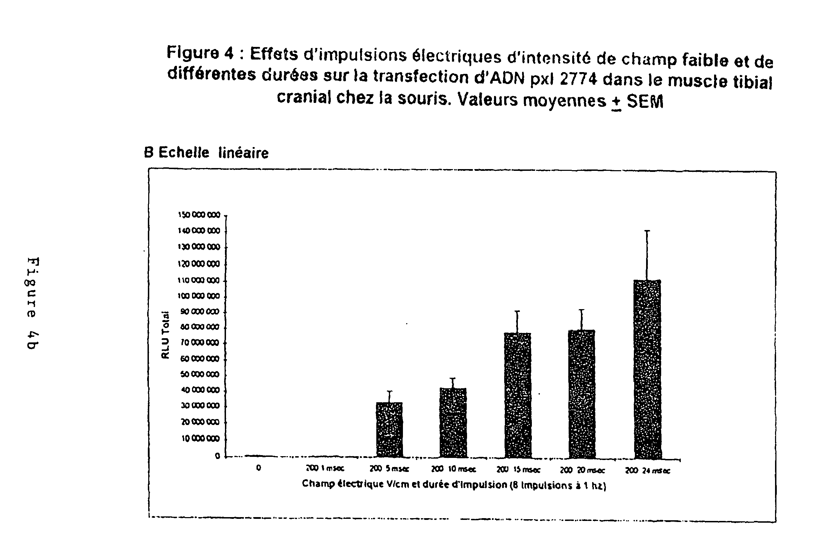

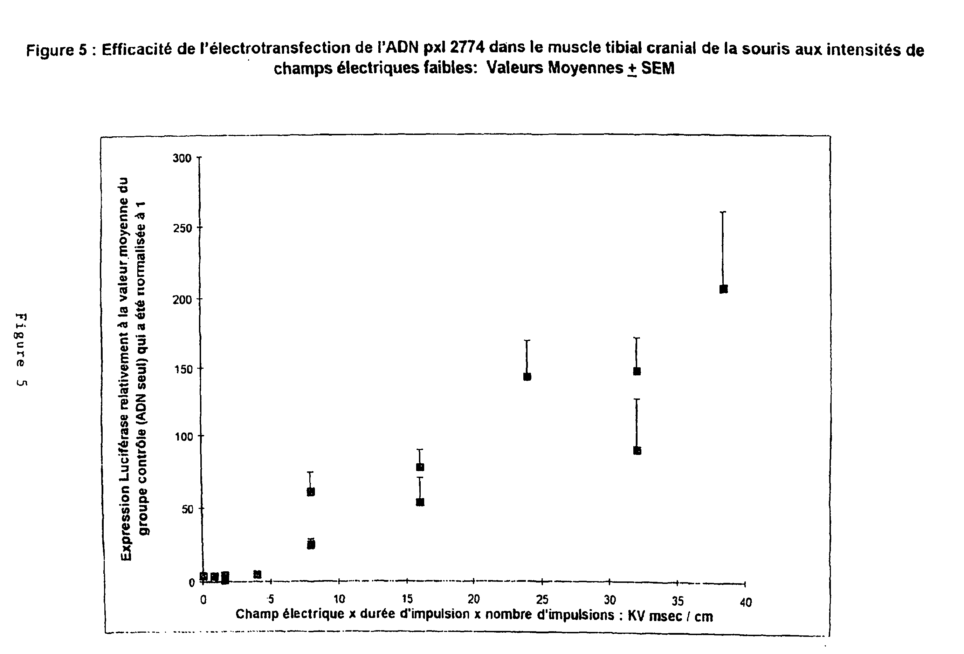

Cet exemple confirme qu'à 200 Volts/cm la transfection de l'activité luciférase est augmentée à partir d'une durée d'impulsion de 5 msec puis continue à croître pour des durées plus longues (figures 4 et 5). Là encore, on observe avec l'électrotransfection une réduction de la variabilité inter-individuelle indiquée par la SEM par rapport au contrôle non électrotransfecté (la valeur relative de la SEM est égale à 35% pour le contrôle et 25, 22, 16, 18, 16 et 26% pour des séries d'impulsions de 1, 5, 10, 15, 20 et 24 msec respectivement).

Dans la meilleure condition de cette expérience, on améliore par un facteur de 205 l'expression du transgène par rapport au contrôle injecté en l'absence d'impulsions électriques. Il apparaît ainsi que la variation dde la durée de chaque impulsion délivrée est un moyen de moduler l'efficacité du transfert d'acides nucléiques et d'ajuster le niveau d'expression du transgène.

### Exemple 5 : efficacité de l'électrotransfert d'acides nucléiques en fonction du produit « nombre des impulsions x intensité du champ x durée de chaque impulsion »

La figure 5 exemplifie l'importance du paramètre correspondant au produit « nombre des impulsions x intensité du champ x durée de chaque impulsion ». Ce paramètre correspond en fait à l'intégrale en fonction du temps de la fonction qui décrit la variation du champ électrique.

La représentation en figure 5 des résultats obtenus au cours des expériences 2, 3 et 4 avec des intensités de champ électrique de 200 V/cm, 100 V/cm ou en absence des champs électriques montre que l'efficacité de transfection augmente en fonction du produit de la durée totale d'exposition au champ électrique par l'intensité de champ. Un effet de stimulation est obtenu pour une valeur supérieure à 1 kVₓmsec/cm du produit « champ électrique x durée totale des impulsions ». Selon un mode préféré, une stimulation est obtenue pour une valeur supérieure ou égale à 5 kVₓmsec/cm du produit « champ électrique x durée totale des impulsions ».

### Exemple 6 : effet de l'augmentation de la durée des impulsions électriques.

Cet exemple illustre que l'on peut augmenter la durée unitaire des impulsions bien au delà des valeurs testées dans l'exemple 4.

Cette expérience est réalisée avec des souris C57B1/6. Le plasmide utilisé est le plasmide pXL 2774, la quantité d'ADN administrée est de 15 µg. L'électropulsateur utilisé pour délivrer les impulsions électrique d'une durée supérieure à 20 msec est un électropulsateur du commerce (Genetronics, modèle T 820, USA, San Diego, CA). Les impulsions électriques sont de nombre et de durée variable mais d'une intensité de champ constante de 200 Volts/cm ; les autres conditions de cette expérience sont celles décrites dans l'exemple 1. Les résultats sont présentés dans le tableau 1.

**Tableau 1 :**

| valeurs moyennes +/- SEM de l'activité luciférase en millions de RLU par muscle . N = 10 pour chaque groupe. Conditions électrotransfert : intensité de champ 200 V/cm, 8 ou 4 impulsions (durée unitaire variable), fréquence 1 Hz. | | | | | | | | | | |
|---|---|---|---|---|---|---|---|---|---|---|
| durée Impulsion (msec) | 0 | 1 | 5 | 10 | 20 | 30 | 40 | 50 | 60 | 80 |
| Expérience A 8 impulsions | 11 ± 5 | 39 ± 6 | 211 ± 26 | 288 ± 46 | 1158 ± 238 | 1487 ± 421 | 2386 ± 278 | | | |
| Expérience A 4 impulsions | 11 ± 5 | 26,8 ± 6 | 123 ± 17 | 246 ± 32 | 575 ± 88 | 704 ± 130 | | 3440 ± 1077 | | |
| Expérience B 4 impulsions | 15 ± 8 | | | | | | 2885 ± 644 | | 2626 ± 441 | 1258 ± 309 |

On constate une augmentation de l'expression du transgène avec l'allongement de la durée unitaire des impulsions (au moins jusqu'à 40 msec pour une série de 8 impulsions et au moins jusqu'à 50 msec pour une série de 4 impulsions d'une intensité de 200 Volts/cm). Cet exemple montre que l'optimum de la durée des impulsions dépend du nombre d'impulsions utilisées et que la durée unitaire des impulsions peut atteindre au moins 80 msec, cette valeur de durée n'étant pas limitative.

### Exemple 7 : efficacité de l'électrotransfert en fonction du nombre d'impulsions électriques

Cette exemple met en évidence l'effet de l'augmentation du nombre d'impulsions électriques sur l'efficacité du transfert d'acides nucléiques.

Cette expérience est réalisée avec des souris C57B1/6. Le plasmide utilisé est le plasmide pXL 2774, la quantité d'ADN administrée est de 15 µg. Les impulsions électriques sont variables en nombre. La durée de chaque impulsion est de 20 msec. L'intensité de champ est de 200 Volts/cm. Les autres conditions de cette expérience sont celles décrites dans l'exemple 1. Les résultats sont présentés dans le tableau 2.

**Tableau 2 :**

| valeurs moyennes +/- SEM de l'activité luciférase en millions RLU par muscle. N = 10 par groupe. Conditions : intensité de champ 200 V/cm, nombre variable d'impulsions de 20 msec, fréquence 1 Hz. | | | | | | | | |
|---|---|---|---|---|---|---|---|---|
| Nombre impulsions | 0 | 1 | 2 | 4 | 6 | 8 | 12 | 16 |
| RLU total | 70 ± 56 | 147 ± 26 | 281 ± 46 | 439 ± 50 | 678 ± 129 | 819 ± 73 | 929 ± 169 | 890 ± 137 |

On observe que l'expression de la luciférase augmente de manière très importante dès l'application d'une seule impulsion, et qu'elle continue d'augmenter en fonction du nombre d'impulsions. Il apparaît ainsi que la variation du nombre d'impulsions délivrées est un moyen de moduler l'efficacité du transfert d'acides nucléiques et d'ajuster le niveau d'expression du transgène.

On confirme également une diminution de la variabilité de la réponse mise en évidence par la diminution de la valeur de la SEM par rapport à la moyenne pour tous les groupes soumis à l'électrotransfert.

### Exemple 8 : effet de l'augmentation de la fréquence des impulsions électriques.

Cet exemple montre que l'augmentation de la fréquence des impulsions permet de manière inattendue d'améliorer l'efficacité de la transfection. D'autre part et dans une perspective clinique, l'augmentation de la fréquence doit améliorer le confort du patient en diminuant la durée totale du traitement.

Cette expérience est réalisée avec des souris C57B1/6. Le plasmide utilisé est le plasmide pXL 2774, la quantité d'ADN administrée est de 15 µg. La fréquence des impulsions électriques est variable (de 0,1 à 4 Hertz). La durée de chaque impulsion est de 20 msec, l'intensité de champ est de 200 Volts/cm, les autres conditions de cette expérience sont celles décrites dans l'exemple 1. Les résultats sont présentés dans le tableau 3.

**Tableau 3 :**

| valeurs moyennes +/- SEM de l'activité luciférase en millions de RLU par muscle. N = 10 pour chaque groupe. Conditions : intensité de champ 200 V/cm, 8 ou 4 impulsions de 20 msec. , fréquence variable. | | | | | | | | |
|---|---|---|---|---|---|---|---|---|
| Fréquence Hertz | 0 | 0,1 | 0,2 | 0,5 | 1 | 2 | 3 | 4 |
| Expérience A 8 impulsions | 5 ± 2 | 54 ± 13 | 95 ± 16 | 405 ± 60 | 996 ± 156 | 1528 ± 257 | | |
| Expérience A 4 impulsions | | 114 ± 14 | 163 ± 24 | 175 ± 26 | 337 ± 53 | 587 ± 90 | | |
| Expérience B 8 impulsions | 21 ± 14 | | | | 1294 ± 189 | 2141 ± 387 | 3634 ± 868 | 2819 ± 493 |
| Expérience B 4 impulsions | | | | | 1451 ± 228 | 1572 ± 320 | 1222 ± 126 | 2474 ± 646 |

Les résultats obtenus dans l'expérience « A » , tableau 3 montrent que les fréquences plus élevées (≥1 Hz) sont plus efficaces que les fréquences faibles qui correspondent à une durée plus longue entre deux impulsions consécutives (10 secondes à 0.1 Hertz). L'efficacité de la transfection augmente avec la fréquence sur la plage de valeurs testées de 0.1 à 4 Hertz pour 4 impulsions et de 0.1 à 3 Hertz pour 8 impulsions.

### Exemple 9 : effet de l'application d'un champ électrique variant selon une exponentielle décroissante en fonction du temps.

Cette exemple met en évidence l'effet de l'application d'un champ électrique variant selon une exponentielle décroissante sur l'efficacité du transfert d'acides nucléiques.

Cette expérience est réalisée avec des souris C57B1/6.

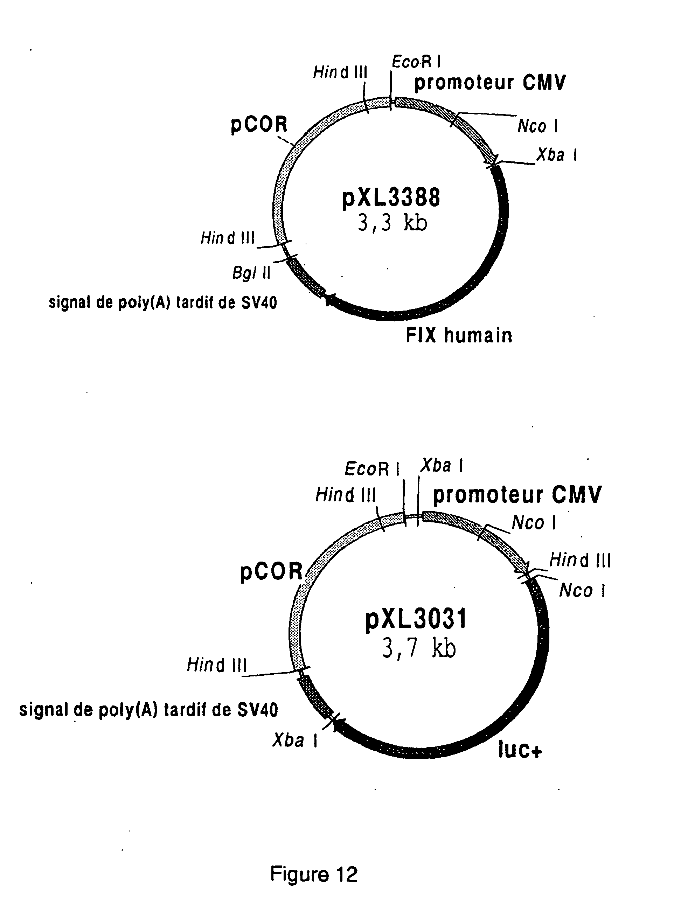

Le plasmide utilisé est le plasmide pXL 3031. Le plasmide pXL3031 (Figure 12) est un vecteur dérivé du plasmide pXL2774 (WO97/10343) dans lequel le gène luc+ codant pour la luciférase de Photinus pyralis modifiée (cytoplasmique) provenant de pGL3basic (Genbank: CVU47295) a été introduit sous contrôle du promoteur provenant de la région précoce du cytomegalovirus humain (hCMV IE, Genbank HS5IEE) et du signal de poly-adénylation de la région tardive du virus SV40 (Genbank SV4CG). La quantité d'ADN administrée est de 10 µg.

Le générateur d'impulsions électriques utilisé permet de délivrer des impulsions d'une intensité de champ électrique variant selon une exponentielle décroissante en fonction du temps (électropulsateur Equibio, modèle easyjectT plus, Kent UK). Le voltage imposé est le voltage au pic de l'exponentielle. Le deuxième paramètre ajustable est la capacitance (µFarads) qui permet de faire varier la quantité d'énergie délivrée et la constante de temps de l'exponentielle. Les résultats sont présentés dans le tableau 4.

**Tableau 4 :**

| facteur d'augmentation de l'expression (activité luciférase) obtenu par application d'une impulsion à décroissance exponentielle. Le facteur d'augmentation est calculé par référence à l'activité luciférase obtenue avec l'administration du plasmide pXL3031 sans électrotransfert. (valeurs moyennes du facteur d'augmentation, N = 4 à 6 par condition). | | | | | | | |
|---|---|---|---|---|---|---|---|
| | Capa µF 150 | Capa µF 300 | Capa µF 450 | Capa µF 600 | Capa µF 1200 | Capa µF 2400 | Capa µF 3000 |
| 40 V/cm | | | | | | 1,23 | 11 |
| 100 V/cm | | | | 16,5 | 2,8 | 6,5 | 23,9 |
| 150 V/cm | | | | 1,8 | 3,5 | 6,1 | |
| 200 V/cm | | 5,1 | | 15,8 | 18,8 | 121,5 | 189,7 |
| 300 V/cm | 32,1 | 90,5 | 48,7 | 760,4 | 56,2 | | |
| 400 V/cm | | 795 | | | | | |
| 600 V/cm | 62 | | | | | | |
| 800 V/cm | 3,1 | 1,1 | | | | | |

A titre comparatif, le facteur d'augmentation de l'expression obtenu pour le transfert de pXL3031 en présence d'un champ électrique avec des impulsions de formes carrées (intensité de champ de 200 V/cm, 8 impulsions de 20 msec, à une fréquence de 1 Hertz) était de 44 dans la même expérience.

Ces résultats montrent que l'on peut utiliser des impulsions électriques de forme carrée ou d'une intensité décroissant de manière exponentielle en fonction du temps. De plus, dans ce dernier cas, une augmentation importante de l'expression peut être obtenue pour une valeur de champ faible et une capacitance élevée (*e.g.* 200 V/cm, capacitance 3000 µFarad) ou une valeur de champ élevée et une capacitance faible (*e.g.* 400 V/cm, capacitance 300 µFarad).

### Exemple 10 : effet de la combinaison d'une impulsion brève de voltage élevé et de plusieurs impulsions longues de voltage faible.

Cet exemple montre que le champ électrique délivré peut être une combinaison d'au moins un champ compris entre 500 et 800 Volts/cm pendant une courte durée, par exemple 50 ou 100 µsec, et d'au moins un champ faible ( < 100Volts/cm) pendant une durée plus longue, par exemple ≥ 1 msec et jusqu'à 90 msec dans cette expérience.

Les valeurs de champ électrique faible sont ici de 80 V/cm appliquées en 4 impulsions d'une durée de 90 msec avec une fréquence de 1 Hertz. Pour cette expérience deux électropulsateurs sont utilisés. Les impulsions électriques sont appliquées par l'un puis l'autre appareil, le changement s'effectuant en moins d'une seconde à l'aide d'une commande manuelle.

Le plasmide utilisé est le plasmide pXL3031. La quantité d'ADN administrée est de 3 µg. Les valeurs de champ électrique sont indiquées dans le tableau 5 ; les autres conditions de cette expérience sont celles décrites dans l'exemple 1.

**Tableau 5 :**

| valeurs moyenne +/- SEM de l'activité luciférase en millions de RLU par muscle. N = 10 muscles par groupe. | | |
|---|---|---|
| Conditions d'application du champ électrique | Expérience 1 (3 µg pXL3031) | Expérience 2 (3 µg pXL3031) |
| Contrôle (absence de champ électrique) | 320 +/-126 | 75 +/- 27 |
| A1 : 500 V/cm , 1 x 0,1 msec | - | 169 +/- 63 |
| A3 : 800 V/cm , 1 x 0,1 msec | 416 +/- 143 | 272 +/- 84 |
| B : 80 V/cm , 4 x 90 msec | 1282 +/- 203 | 362,21 +/- 85,17 |
| Conditions A1 puis B | - | 1479 +/- 276 |
| Conditions A3 puis B | 3991 +/- 418 | 1426 +/- 209 |
| Conditions B puis A3 | - | 347 +/- 66 |

Le tableau 5, résumant les résultats obtenus pour deux séries d'expériences, montre qu'une brève impulsion de voltage élevé ou que quatre impulsions successives longues et de faible voltage améliorent peu la transfection relativement au groupe contrôle ayant reçu une injection de pXL3031 mais non soumis à un champ électrique. Il en est de même lorsque les impulsions de champ faible sont appliquées avant l'impulsion de champ élevé.

Par contre, dans les deux séries d'expériences, la combinaison d'une brève impulsion de haut voltage suivie de quatre impulsions successives longues et de faible voltage augmente très nettement l'efficacité du transfert de l'ADN.

Les résultats obtenus dans les exemples 1 et 2 ont montré que 8 impulsions de 600, 800 ou 1200 volts d'une durée unitaire de 1 msec à 1 Hz étaient lésionnelles et inhibaient la transfection. Les résultats obtenus dans l'exemple 10 montrent que, dans des conditions particulières, il est possible d'utiliser des intensités de champ de voltage élevées de façon non lésionnelle, en effet d'un point de vu macroscopique les muscles ne sont jamais visiblement altérés. L'utilisation de champs électriques élevés de durée brève combinés à des champs faibles de durée plus longue apparaît comme un moyen supplémentaire de moduler l'efficacité du transfert de l'ADN.

### Exemple 11 : électrotransfert avec des plasmides de tailles différentes, des gènes sous contrôle de différents promoteurs ou avec des sites d'adressage variable de la protéine exprimée par le transgène.

### 11. a - électrotransfert avec des plasmides de tailles différentes

Des plasmides de taille différente (2.8 Kb, 3.8 Kb, 8.6 Kb, 20 Kb, et 52,5 Kb) comprenant le gène codant pour la luciférase ont été testés. La quantité de plasmide administrée est de 10 µg par muscle. Un champ électrique d'une intensité de 200 V/cm en 8 impulsions de 20 msec à 2 Hz est appliqué , les autres conditions de cette expérience étant celles décrites dans l'exemple 1.

On observe une augmentation de l'expression du transgène d'environ 50 fois avec les plasmides de 2,8 Kb et 3,8 Kb, d'environ 80 fois avec le plasmide de 8,6 Kb et de 3 à 6 fois avec les plasmides de 20 et 52,6 Kb.

Cet exemple démontre ainsi la possibilité de transférer des plasmides de taille importante, allant jusqu'à 20 Kb et au delà.

### 11. b : contrôle du signal de luminescence en absence de gène codant pour la luciférase.

A titre de contrôle, et pour exclure la possibilité que les signaux de luminescence observés pour le dosage de l'activité luciférase soient dûs à des radicaux produits dans le tissu suite au traitement électrique, l'activité luciférase a été testée sur des muscles traités avec un plasmide ne codant pas pour la luciférase et soumis à un champ électrique.

**Tableau 6 :**

| activité luciférase dans des muscles injectés avec différents plasmides, avec ou sans application d'un champ électrique. Conditions : 200 V/cm, 8 impulsions de 20 msec, fréquence 1 Hz. Valeurs moyennes +/- SEM de l'activité luciférase en millions de RLU par muscle. | | |
|---|---|---|
| Electrotransfert | - | + |
| Plasmide pXL 3004 ( 15 µg) codant pour la β-galactosidase | 0,016 +/- 0,005 (n=6) | 0,015 +/- 0,006 (n=6) |
| Plasmide pXL 2774 (15µg) codant pour la luciférase | 7,33 +/- 3,53 (n=10) | 491,71 +/- 122,28 (n=10) |

Les résultats montrent que l'activité basale de la luciférase dans des muscles injectés avec un plasmide ne codant pas pour la luciférase est tout a fait négligeable.

### 11. c - électrotransfert de gènes sous contrôle de différents promoteurs.

L'influence de différents promoteurs a été testée sur le niveau d'expression des gènes transférés, avec et sans, application du champ électrique.

La quantité de plasmide injecté par muscle est de 2 µg. Le champ électrique appliqué est de 200 V/cm en 8 impulsions de 20 msec à 1 Hz , les autres conditions de cette expérience sont celles décrites dans l'exemple 1. Les résultats sont présentés dans le tableau 7. Le plasmide testé est le plasmide pXL3031 pour la construction CMV-LUC. La construction PGK correspond à la substitution du promoteur CMV par le promoteur PGK dans le pXL3031.

**Tableau 7 :**

| valeurs moyennes +/- SEM de l'activité luciférase en millions de RLU par muscle. | | | | |
|---|---|---|---|---|
| Promoteur | PGK | | CMV | |
| Electrotransfert | - | + | - | + |
| RLU | 8 ± 2,8 | 1070 ± 327 | 157 ± 83 | 20350 ± 1112 |
| Facteur d'amplification | x 133,7 | | x 129,3 | |

Ces résultats montrent que, lorsque l'ADN est transféré en présence d'un champ électrique, le facteur d'augmentation de l'expression du transgène est comparable quelle que soit l'origine ou la force du promoteur.

### 11. d - électrotransfert de gène avec différents sites d'adressage de la protéine exprimée par le transgène.

Cet exemple illustre le transfert de gène codant pour des protéines ayant différentes localisations cellulaires. Le plasmide pXL3031 code pour une luciférase synthétisée dans le cytosol et le plasmide pXL2774 code pour une luciférase adressée dans les peroxysomes.

La quantité de plasmide injectée par muscle est de 10 µg. Le champ électrique appliqué est de 200 V/cm en 8 impulsions de 20 msec à 1 Hz , les autres conditions de cette expérience sont celles décrites dans l'exemple 1. Les résultats sont présentés dans le tableau 8.

**Tableau 8 :**

| valeurs moyennes +/- SEM de l'activité luciférase en millions de RLU. | | | |
|---|---|---|---|
| pXL 2774 | | pXL 3031 | |
| Electrotransfert - | Electrotransfert + | Electrotransfert - | Electrotransfert + |
| 11 ± 5 | 1158 ± 238 | 839 ± 281 | 111524 ± 16862 |

Ces résultats mettent en évidence que le procédé selon l'invention s'applique pour le transfert de gènes codant pour des protéines de localisations cellulaires différentes, et notamment pour des protéines péroxisomales ou des protéines cytosoliques.

### Exemple 12 : analyse cinétique et histologique de l'expression du transgène.

### 12. a - cinétique d'expression du transgène

Cet exemple montre que le transfert d'acides nucléiques en présence d'un champ électrique dans les conditions selon l'invention permet d'obtenir l'expression d'un transgène à un niveau élevé et stable pendant une durée d'au moins 4 mois.

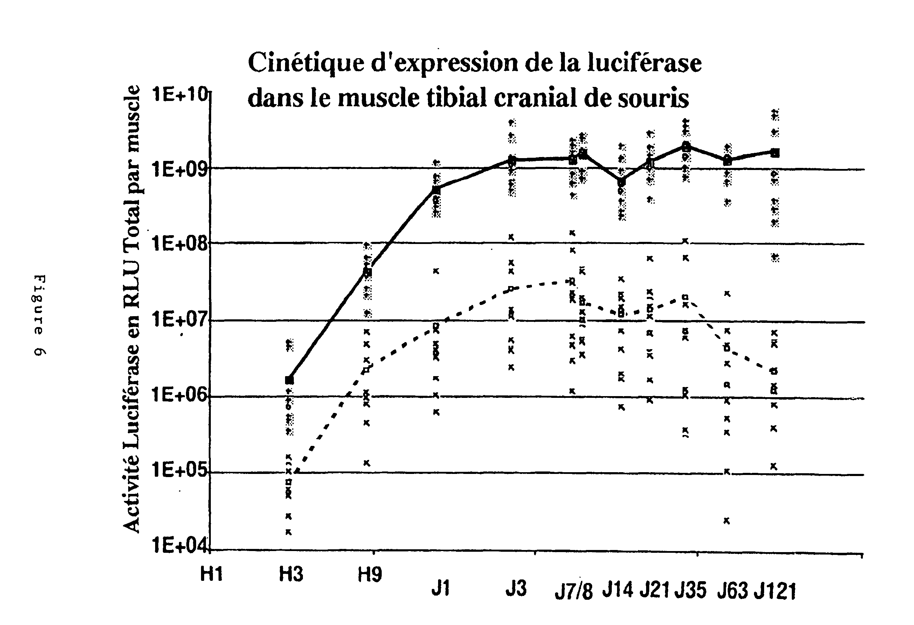

Cette expérience est réalisée avec des souris C57B1/6. Le plasmide utilisé est le plasmide pXL 2774, la quantité d'ADN administrée est de 15 µg. L'injection d'ADN est suivie, ou non (groupe contrôle), de l'application d'un champ électrique dans les conditions suivantes : intensité 200 V/cm , 8 impulsions de 20 msec, fréquence 1 Hz. Les autres conditions de cette expérience sont celles décrites dans l'exemple 1. L'activité luciférase est déterminée sur des groupes de 10 souris sacrifiées à différents temps. Les résultats sont présentés dans la figure 6.

On observe, pour le groupe contrôle, que l'expression de la luciférase est détectable dès la 3 ème heure après l'injection du plasmide et augmente jusqu'au 3 ème jour (J3) puis décroît de manière notable après 35 jours.

On constate, pour le groupe soumis aux impulsions électriques, que l'expression du transgène se maintient à un niveau très nettement supérieur à celui du groupe contrôle. De plus, et de manière remarquable, on observe que ce niveau d'expression reste élevé et constant au delà de 35 jours et au moins jusqu'à 120 jours. Ce niveau d'expression élevé et durable du transgène est un résultat particulièrement avantageux dans la perspective de traitements cliniques à long terme avec des gènes thérapeutiques.

### 12. b - analyse histologique

Une étude histologique a été conduite dans les mêmes conditions mais en administrant le plasmide pCOR CMV-lacZ (pXL3004) codant pour la β-galactosidase à localisation nucléaire.

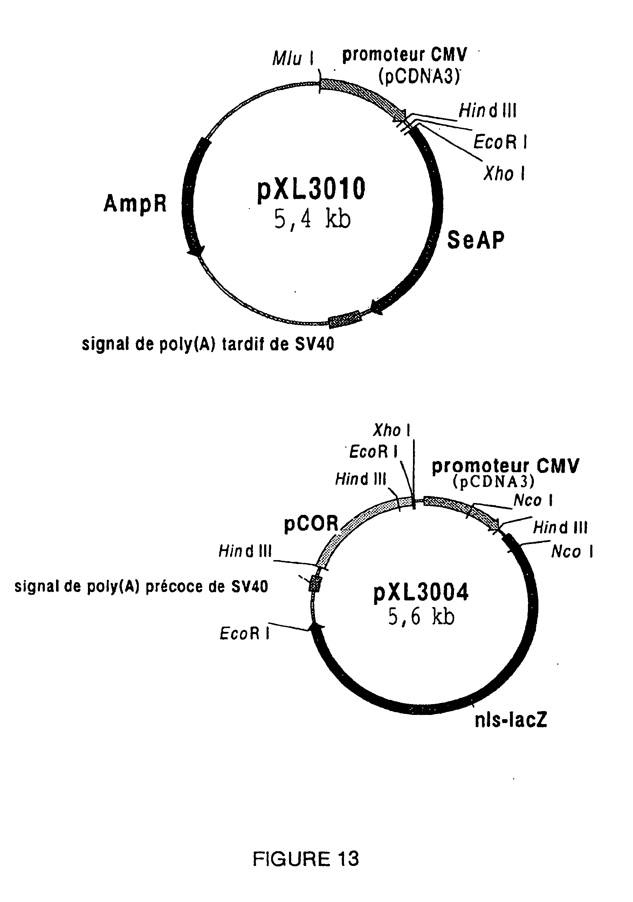

Le plasmide pXL3004 (Figure 13) est un vecteur dérivé du plasmide pXL2774 (WO97/10343) dans lequel le gène lacZ additionné d'une séquence de localisation nucléaire (nls) (Nouvel et al., 1994, Virology 204:180-189)) a été introduit sous contrôle du promoteur CMV du plasmide pCDNA3 (Invitrogen, Pays-Bas) et du signal de poly-adénylation de la région précoce du virus SV40 (Genbank SV4CG).

Les animaux sont sacrifiés sept jours après administration du plasmide. L'analyse histologique permet de détecter les cellules exprimant la β-galactosidase et dont le noyau est situé dans le plan de coupe (histochimie Xgal).

Le nombre de fibres musculaires présentant des noyaux positifs au niveau des coupes examinées est en moyenne de 76 dans le groupe (n=8) ayant reçu le plasmide pXL3004 puis soumis aux impulsions électriques contre une moyenne de 8.5 dans le groupe contrôle (n=8) (animaux ayant reçu le plasmide pXL3004 mais n'ayant pas été soumis aux impulsions électriques).

On observe que le nombre de fibres musculaires exprimant le transgène est en moyenne neuf fois plus élevé par rapport au groupe contrôle. La plupart de ces fibres musculaires sont quiescentes avec des noyaux situés en périphérie. De très rares fibres musculaires centronuclées expriment la β-galactosidase. On observe également que, le long du trajet d'injection du plasmide, la densité de fibres musculaires positives par unité de surface est plus importante dans le groupe traité par électrotransfert par rapport au groupe contrôle.

L'ensemble de ces résultats montrent que, relativement à des muscles non soumis au champ électrique, l'électrotransfert permet une très nette augmentation du nombre de fibres musculaires exprimant le transgène ainsi qu'une très nette augmentation de la surface de la zone exprimant le transgène. Il est observé également que l'application du champ électrique n'entraîne pas de réaction inflammatoire notable.

### Exemple 13 : Effet du moment d'injection de l'acide nucléique par rapport à l'application du champ électrique.

Cet exemple illustre le fait que l'acide nucléique peut être administré au moins 30 minutes, et même au moins une heure, avant l'application du champ électrique.

Cette expérience est réalisée avec des souris C57B1/6. Le plasmide utilisé est le plasmide pXL 2774. La quantité d'ADN administrée est de 15 µg ou 1,5 µg. L'injection d'ADN est suivie, ou précédée, de l'application d'un champ électrique dans les conditions suivantes : intensité 200 V/cm , 8 impulsions de 20 msec, fréquence 1 Hz. Les autres conditions de cette expérience sont celles décrites dans l'exemple 1. Un groupe contrôle est constitué d'animaux ayant reçu une injection du plasmide mais n'ayant pas été soumis aux impulsions électriques. Les résultats sont présentés dans le tableau 9

**Tableau 9:**

| valeurs moyennes +/- SEM de l'activité luciférase en millions de RLU par muscle. N = 10 muscles par groupe. | | | | | |
|---|---|---|---|---|---|
| Tableau 9 A : Injection ADN en absence de champ électrique | | | | | |
| | **Exp 1** | **Exp 2** | **Exp 3** | **Exp 4** | **Exp 5** |
| | pXL2774 (15 µg) | pXL 2774 15 µg) | pXL 2774 (1,5 µg) | pXL 2774 (15 µg) | pXL 2774 (1,5 µg) |
| Contrôle | 7 ± 4 | 8 ± 6 | 0,4 ± 0,2 | 22 ± 15 | 1 ± 1 |

| Tableau 9 B : Injection ADN avant application du champ électrique | | | | | |
|---|---|---|---|---|---|
| **temps** | **Exp 1** | **Exp 2** | **Exp 3** | **Exp 4** | **Exp 5** |
| - 120 min | | | | 20 ± 5 | 2 ± 1 |
| - 60 min | | | | 106 ± 22 | 10 ± 3 |
| - 30 min | 303 ± 36 | 237 ± 61 | 7 ± 3 | 184 ± 22 | 15 ± 4 |
| - 5 min | 410 ± 7 | | | | |
| - 60 sec | 253 ± 51 | | | | |
| - 20 sec | 492 ± 122 | 201 ± 43 | 9 ± 3 | 123 ± 23 | 12 ± 2 |

| Tableau 9 C : Injection ADN après application du champ électrique | | | | | |
|---|---|---|---|---|---|
| **temps** | **Exp 1** | **Exp 2** | **Exp 3** | **Exp 4** | **Exp 5** |
| + 10 sec | | | | 7 ± 7 | |
| + 20 sec | 11 ± 6 | 0,4 ± 0,1 | | | |
| + 60 sec | 8 ± 7 | | | 17 ± 15 | |

La présence de l'ADN au moment de l'application du champ électrique est une condition de l'efficacité de l'électrotransfection. De façon remarquable, il est observé que l'injection du plasmide peut être réalisée au moins 30 minute et même 1 heure (expériences 4 et 5) avant l'application du champ électrique et ce, sans modification notable du niveau d'expression. Un résultat similaire est obtenu aussi bien avec avec une dose de 15 µg de plasmide par muscle qu'avec une dose 10 fois plus faible de 1,5 µg.

Ces observations permettent notamment d'envisager de multiples injections à des temps variables du même plasmide, ou de différents plasmides, dans le muscle préalablement à l'application du champ électrique. Il est également possible de faire de multiples injections sur une zone étendue du muscle puis d'appliquer une série d'impulsions électriques sur l'ensemble du territoire injecté à traiter.

### Exemple 14: Etude statistique sur la relation entre la dose d'ADN injecté et le niveau d'expression.

L'étude statistique présentée dans cet exemple permet de comparer la relation effet/dose d'un transgène administré en présence, ou en absence, d'un champ électrique. Cette étude confirme également que le procédé selon l'invention réduit considérablement la variabilité d'expression du transgène.

Des souris C57B16 âgées de 5 semaines ont reçu une injection de plasmide pXL3031 dans le muscle tibial cranial et de manière bilatérale. Les doses de plasmide varient de 0,25 à 32µg d'ADN. Chaque dose est testée sur 10 animaux. Immédiatement après l'injection du plasmide, on soumet l'une des deux pattes à un champ de 250V/cm, avec 4 impulsions de 20ms et une fréquence de 1 Hz.

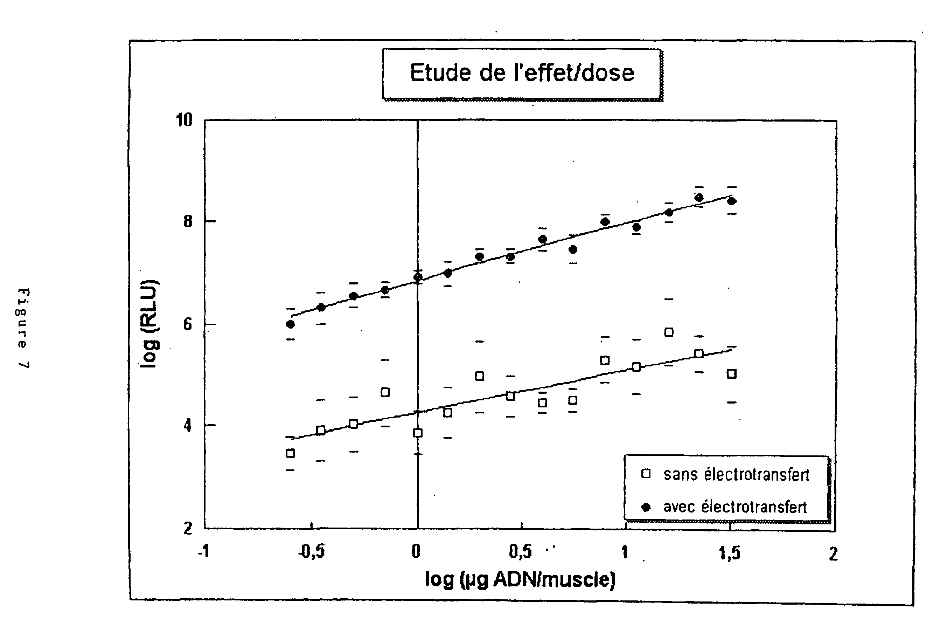

Les animaux sont sacrifiés 5 jours après le traitement et l'expression du transgène est recherchée dans l'extrait tissulaire de chaque muscle. Les résultats sont présentés dans la figure 7.

La comparaison de l'évolution des variances en fonction de celle des moyennes pour chaque série de 10 répétitions montre clairement que la distribution de l'expression du transgène est log-normale. L'analyse graphique des résultats de la figure 7, confirmée par le calcul, montre que l'expression varie linéairement avec le logarithme de la dose d'ADN injecté.

Le test de Cochran montre qu'il existe une homogénéité des variances pour chaque régression (avec et sans électrotransfert) ce qui permet d'utiliser les variances résiduelles pour effectuer l'ensemble des calculs.

Un test d'écart à la linéarité est non significatif au risque 5% dans le cas où il y a eu électrotransfert, par contre il existe un écart à la linéarité très significatif (p < 0,01), ce qui traduit une importante hétérogénéité des réponses en l'absence d'électrotransfert. La variance résiduelle est 5 fois plus faible avec l'électrotransfert.

Compte tenu des valeurs estimées des variances résiduelles, il est possible d'utiliser 5 fois moins d'animaux pour obtenir la même puissance dans un test de comparaison d'efficacité de transfection, selon que l'on applique ou non l'électrotransfert. Ainsi pour mettre en évidence une différence d'expression d'un facteur 2, 5 ou 10, avec un intervalle de confiance P=95%, il faudra respectivement 33, 8 ou 5 animaux si le transgène est administré par électrotransfert et 165, 40 ou 25 animaux en absence d'électrotransfert. Un tableau est présenté ci-dessous résumant ce type de calcul dans le cas où l'électrotransfert est utilisé.

| Rapport d'efficacité ou d'expression | P = 95% | P = 90% | P = 85% | P =75% |
|---|---|---|---|---|
| 2 | 33 | 28 | 24 | 19 |
| 5 | 8 | 7 | 6 | 6 |
| 10 | 5 | 5 | 4 | 4 |

Ainsi la diminution de la variabilité interindividuelle obtenue avec l'électrotransfert permet d'effectuer des études analytiques précises sur la comparaison de l'expression de différents gènes. Elle autorise également une meilleure définition des doses de traitement et doit prévenir le risque lié au dépassement des doses acceptables dans la fenêtre thérapeutique.

Le test de comparaison des pentes obtenues pour chaque régression est non significatif. On peut donc considérer au risque de 5% qu'il y a parallélisme des deux régressions.

Le calcul de la puissance relative montre que pour atteindre un niveau d'expression comparable à celui obtenu en présence d'électrotransfert il faut, en absence d'électrotransfert, environ 250 fois plus d'ADN injecté par muscle (243 +/- 85 ; intervalle de confiance P = 95%).

Le calcul de la puissance relative montre corrélativement que, pour une quantité d'ADN donnée, le niveau d'expression est environ 500 fois plus élevé en présence d'électrotransfert par comparaison au niveau d'expression obtenu en l'absence d'électrotransfert.

### Exemple 15 : comparaison de différents types d'électrodes.

Cet exemple a pour but de comparer l'effet de deux types d'électrodes, électrodes plaques et électrodes aiguilles, sur l'efficacité du transfert d'acides nucléiques. Les électrodes aiguilles ont également été testées selon différentes orientations d'implantation.

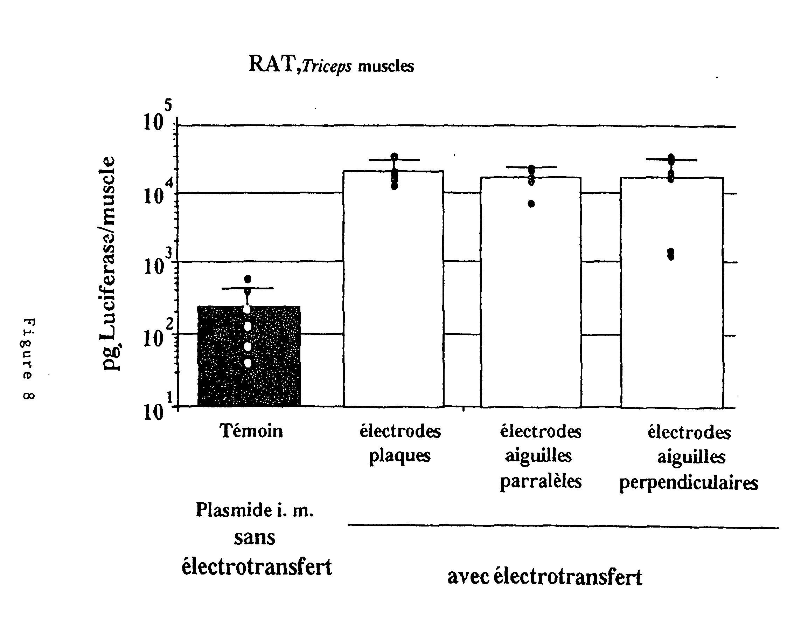

La plasmide pXL 2774 (150 µg) est injecté dans le muscle triceps chez le rat. Les électrodes plaques sont placées comme indiqué dans l'exemple 1. La distance inter-électrode pour les électrodes plaques est de 1,2 cm. Pour les électrodes aiguilles, la distance inter-électrodes est de 0,9 cm. Les électrodes aiguille sont enfoncées dans le tissu musculaire sur une longueur équivalente, soit perpendiculairement, soit parallèlement à l'axe des fibres, de part et d'autre du site d'injection. Quel que soit le type d'électrodes, ou leur orientation, les conditions d'application du champ électrique sont les suivantes : intensité 200 V/cm , 8 impulsions de 20 msec à 2 Hz. Les résultats sont présentés dans la figure 8.

Les résultats obtenus montrent que l'application du champ électrique à l'aide de deux aiguilles parallèles implantées dans le muscle donne des résultats comparables à ce qui est obtenu avec des électrodes plaques mises au contact de la peau entourant le muscle. Il est également montré que l'efficacité de l'électrotransfert est indépendante de la direction d'implantation des électrodes aiguilles relativement à l'axe des fibres musculaires.

Cet exemple montre que le procédé selon l'invention permet l'électrotransfert d'acides nucléiques à l'aide d'électrodes externes ou invasives et ce, quelle que soit leur orientation. L'utilisation des électrodes aiguilles est particulièrement avantageuse pour assurer le transfert d'acides nucléiques dans les muscles de grande taille tout en conservant des impulsions électriques de voltage modéré (par exemple 100 V avec un espacement de 0,5 cm pour délivrer un champ électrique de 200 V/cm)

### Exemple 16 : efficacité de l'électrotransfert sur différents muscles de la souris, du rat, du lapin et du singe.

Cet exemple illustre que l'électrotransfert d'acides nucléiques est applicable à différents types de muscles, dans différentes espèces de mammifères (souris, lapin, rat et singe).

Les conditions d'application du champ électrique sont définies dans le tableau 10 A en regard de chaque espèce. Les résultats sont présentés dans le tableau 10 A.

**Tableau 10 A :**

| facteur d'augmentation de l'expression de la luciférase obtenu avec l'électrotransfection. Ce facteur est calculé par référence à l'activité luciférase obtenue pour l'injection du plasmide pXL3031 ou pXL2774 sans électrotransfert. Moyennes sur 10 muscles par groupe. L'activité luciférase est dosée 7 jours après l'administration du plasmide. | | | | | | | |
|---|---|---|---|---|---|---|---|
| Espèce | plasmide | impulsions électriques | Muscle Tibial cranial | Muscle Gastroc-némien | Muscle Rectus femoris | Muscle Triceps brachii | Muscle Quadriceps |
| Souris | 10 µg pXL3031 | 8 x 200 V/cm 20 msec, 2 Hz | x 28 | x 196 | x 342 | x 1121 | |
| Rat | 150 µg pXL3031 | 8 x 200 V/cm 20 msec, 2 Hz | x 31 | | | x 160 | x 13,2 |
| Lapin | 200 µg pXL2774 | 4 x 200 V/cm 20 msec, 1 Hz | x 25417 | | | x 724 | x 3595 |

L'électrotransfert a également été testé chez le singe (*Macaca fascicularis*). Le plasmide utilisé est le plasmide pXL3179 comprenant le gène codant pour le facteur de croissance de fibroblastes 1 (FGF1 ou aFGF).

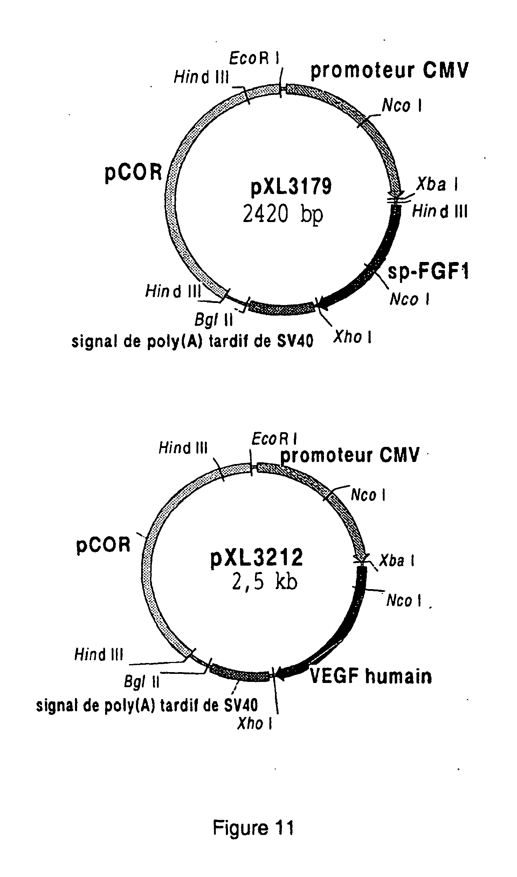

Le plasmide pXL3179 (Figure 11) est un vecteur dérivé du plasmide pXL2774 (WO97/10343) dans lequel le gène codant pour une fusion entre le peptide signal de l'interféron de fibroblastes humain et l'ADNc du FGF1 (Fibroblast Growth Factor1) (sp-FGF1, Jouanneau et al., 1991 PNAS 88:2893-2897) a été introduit sous contrôle du promoteur provenant de la région précoce du cytomegalovirus humain (hCMV IE) et du signal de poly-adénylation de la région tardive du virus SV40 (Genbank SV4CG).

La présence de FGF1 est déterminée par immuno-histochimie. Les valeurs indiquent le nombre de coupes positives 3 jours après injection intra musculaire de 500 µg du plasmide pXL3179. Les conditions d'application du champ électrique sont les suivantes : intensité du champ électrique 200 V/cm , 8 impulsions de 20 msec à 1 Hz. Les résultats sont présentés dans le tableau ci dessous.

**Tableau 10 B :**

| révélation par immuno-histochimie de l'expression de FGF1 dans différents muscles de singe (*Macaca fascicularis*). Les valeurs indiquent le nombre de coupes positives 3 jours après injection intra musculaire de 500 µg du plasmide pXL3179 codant pour FGF1 avec ou sans électrotransfert. | | |
|---|---|---|
| | Electrotransfert | |
| Muscle | - | + |
| Triceps | 0 | 0 |
| Tibial cranial | 0 | 30 |
| Biceps | 0 | 4 |
| Quadriceps | 0 | 30 |

Ces résultats démontrent que l'électrotransfert augmente de manière remarquable l'expression d'un transgène, dans différents types de muscles, dans différentes espèces de mammifères.

### Exemple 17 : efficacité de l'electrotransfert sur le muscle du diaphragme de rat

La possibilité d'exprimer de façon durable et stable des gènes d'intérêt thérapeutique directement au niveau du diaphragme est une approche thérapeutique particulièrement intéressante dans le cadre du traitement de certaines maladies dégénératives qui affectent le fonctionnement de ce muscle, telle que notamment la myopathie de Duchenne.

Les rats sont anesthésiés avec un mélange largactyl, kétamine ( 1 mg/kg largactyl, 150 mg/kg ketamine). Dans ces expériences le diaphragme est rendu accessible par une incision le long du sternum. L'injection est réalisée dans l'hémidiaphragme (50 µg de plasmide pXL 2774 dans 50 µl de NaCl 20 mM et glucose 5 %). Les électrodes plaques sont ensuite placées de part et d'autre du plan du diaphragme le long du trajet d'injection (distance inter électrodes = 1 mm). Les conditions d'électrotransfert sont les suivantes : intensité du champ 160 V/cm ou 300 V/cm, 8 impulsions de 20 msec, fréquence 1 hertz. Le champ électrique est appliqué moins d'une minute après l'injection. L'animal est ensuite recousu.

**Tableau 11 :**

| valeurs moyennes +/- SEM de l'activité luciférase en millions de RLU par muscle. N = 12 pour chaque groupe. | | | |
|---|---|---|---|
| V/cm | 0 | 160 | 300 |
| RLU total | 48 ± 33 | 920 ± 474 | 51 ± 29 |

Cette exemple montre une amélioration significative de l'expression du transgène dans le diaphrame après application de 8 impulsions de 20 msec d'une intensité de champ de 160 V/cm (p < 0,003 avec le test non paramétrique de Mann-Whitney).

### Exemple 18 : transfert d'un gène codant pour la phosphatase alcaline sécrétée (SeAP) et cinétique d'expression de la SeAP.

Cet exemple illustre la capacité du procédé selon l'invention de transformer le muscle en un organe sécréteur d'un polypeptide d'intérêt thérapeutique ou vaccinal et d'assurer la présence dans la circulation sanguine d'une concentration élevée et stable du polypeptide d'intérêt.

Dans cet exemple le procédé d'électrotransfert à été testé sur la souris adulte avec un plasmide comprenant le gène codant pour la phosphatase alcaline sécrétée placentaire humaine. Des souris C57BL6 adultes ont reçu, dans le muscle tibial cranial et de manière unilatérale, une injection de plasmide pXL3010.

Le plasmide pXL3010 (Figure 13) est un vecteur dérivé de ColE1 dans lequel le gène codant pour la phosphatase alcaline sécrétée provenant de pSEAP-basic (Clontech, Genbank: CVU09660) a été introduit sous contrôle du promoteur CMV provenant du plasmide pCDNA3 (Invitrogen, Pays-Bas) et du signal de poly-adénylation de la région tardive du virus SV40 (Genbank SV4CG).

Les conditions d'électrotransfert sont les suivantes : champ électrique 200 V/cm, 8 impulsions de 20 msec, fréquence 1 Hz. Le champ électrique est appliqué 20 secondes après injection. Les prélèvement sanguins sont réalisés 7 jours plus tard au niveau du plexus rétroorbitaire. La concentration en phosphatase alcaline dans le sérum est réalisée est mesurée à l'aide d'un test de chemiluminescence (Phospha-light, Tropix, Bedford, MA 01730). L'injection dans le muscle d'un plasmide non codant (pUC19), suivie ou non de l'application d'un champ électrique, permet de vérifier l'absence de signal correspondant à l'activité phosphatase alcaline endogène. Les résultats sont présentés dans le tableau 12.

**Tableau 12 :**

| valeurs moyennes ± SEM de la concentration de phosphatase alcaline (SeAP) circulante dans le sang en ng/ml de sérum. | | | |
|---|---|---|---|
| Plasmide pXL3010 µg | Plasmide pUC19 µg | Electrotransfert - | Electrotransfert + |
| 0,1 | 0 | 0,03 ± 0,01 (n=5) | 1,23 ± 0,21 (n=10) |
| 0,3 | 0 | 0,05 ± 0,02 (n=5) | 1,92 ± 0,33 (n=10) |
| 1 | 0 | 0,16 ± 0,04 (n=5) | 7,58 ± 1,18 (n=10) |
| 10 | 0 | 1,52 ± 0,59 (n=10) | 262,87 ± 54,97 (n=10) |
| 400 | 0 | 15,64 ± 10,77 (n=5) | 2203,11 ± 332,34(n=5) |
| 0,1 | 9,9 | 0,088 ± 0,015 (n=5) | 21,39 ± 3,54 (n=10) |
| 0,3 | 9,7 | 0,90 ± 0,49 (n=5) | 95,67 ± 16,15 (n=10) |
| 1 | 9 | 0,26 ± 0,09 (n=5) | 201,68 ± 32,38 (n=10) |
| 10 | 0 | 0,21 ± 0,05 (n=10) | 357,84 ± 77,02 (n=10) |

Lorsque le plasmide pXL3010 est administré par électrotransfection, on constate une augmentation d'un facteur 140 ou 170 de la concentration de SeAP dans le sang.

L'injection de 400 µg de plasmide (injection de 100 µg d'ADN dans le muscle tibial cranial en bilatéral et en deux fois à 30 minutes d'intervalle avant application du champ électrique) permet d'atteindre avec l'électrotransfert une concentration sérique de 2200 ng/ml en phosphatase alcaline contre 16 ng/ml en l'absence d'électrotransfert.

Il faut noter que l'ajout d'un ADN non codant (pUC19) qui permet de travailler à quantité d'ADN constante (10 µg d'ADN total par souris) permet encore d'améliorer le niveau d'expression de la phosphatase alcaline pour de faibles quantités de plasmide pXL3010 injectées (≤1µg).

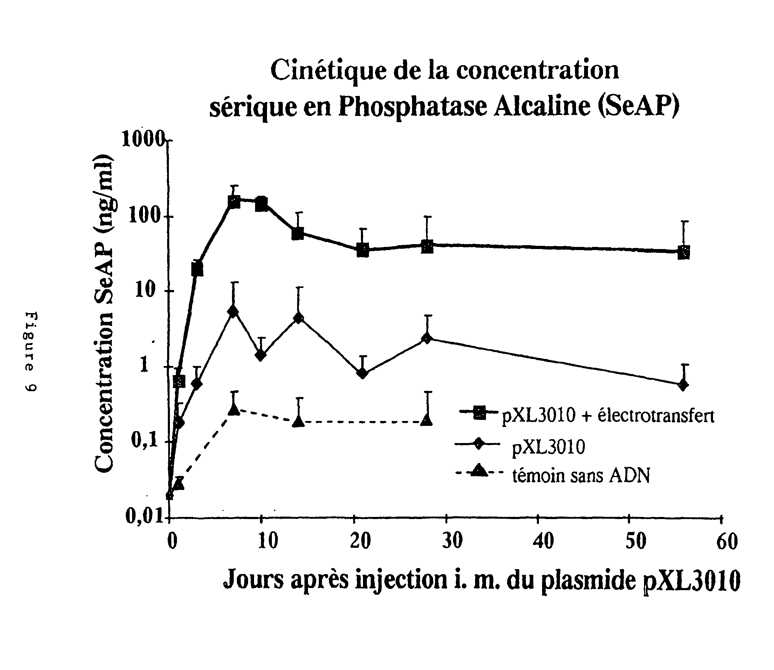

Une cinétique d'expression de la SeAP a été réalisée. La dose de plasmide administrée est de 15 µg par mucle en bilatéral, soit 30 µg par souris. Les résultats sont présentés dans la figure 9. On observe, dès 7 jours après injection, une augmentation importante et durable (au moins pendant 2 mois) de la concentration de SeAP détectée dans le sang lorsque le plasmide pXL3010 est administré par électrotransfert.

L'ensemble de ces résultats confirme que le transfert d'acides nucléiques dans le muscle avec le procédé selon l'invention permet d'obtenir un niveau d'expression élevé et durable, aussi bien pour des protéines localisées dans le muscle que pour des protéines sécrétées et qu'il est ainsi possible de transformer le muscle en un organe secréteur de polypeptide d'intérêt.

### Exemple 19 : transfert d'un gène codant pour l'érythropoiëtine (EPO)

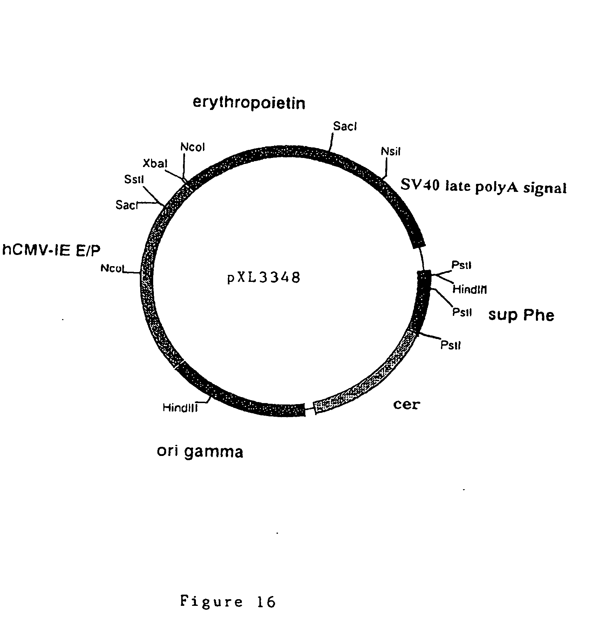

Des souris C57B1/6 adultes ont reçu, dans le muscle tibial cranial et de manière unilatérale, une injection de plasmide pXL3348. Le plasmide pXL3348 (Figure 16) est un vecteur dérivé du plasmide pXL2774 dans lequel le gène murin de l'erythropoïetine (NCBI : 193086) a été introduit sous contrôle du promoteur provenant de la région précoce du cytomegalovirus humain (hCMV IE) et du signal de poly-adénylation de la région tardive du virus SV40 (Genbank SV4CG).

Les conditions d'électrotransfert sont les suivantes : intensité champ électrique 200 V/cm, 8 impulsions de 20 msec, fréquence 1 Hz. Le champ électrique est appliqué immédiatement après injection de l'ADN plasmidique.

**Tableau 13 :**

| valeurs moyennes ± SEM. N = 4 à 5. | | | | |
|---|---|---|---|---|
| | Erythropoïetine sérique (mIU/ml) à J7 | | Erythropoïetine sérique (mIU/ml) à J24 | |
| Plasmide | Electrotransfert - | Electrotransfert + | Electrotransfert - | Electrotransfert + |
| pXL3348 (1 µg) | 0 | 3,0 ± 1,6 | 0 | 1,12 ± 0,8 |
| pXL3348 (10 µg) | 0,9 ± 0,9 | 61,8 ± 15,8 | 0 | 74,1 ± 28,9 |
| pUC19 (1 µg) | | 0 | | 0 |

| | Hématocrite % Prélèvement à J7 | | Hématocrite % Prélèvement à J24 | |
|---|---|---|---|---|
| Plasmide | Electrotransfert - | Electrotransfert + | Electrotransfert - | Electrotransfert + |
| pXL3348 (1 µg) | 38,5 ± 0,5 | 35,0 ± 3,6 | 50,8 ± 2,3 | 81 ± 0,5 |
| pXL3348 (10 µg) | 32,0 ± 3,2 | 26,0 ± 4,1 | 69,0 ± 5,1 | 83,0 ± 1,0 |
| PUC 19 (1 µg) | | 30,8 ± 2,3 | | 43,2 ± 0,9 |

On observe, avec l'électrotransfert, une très nette augmentation de la quantité d'érythropoiëtine dans le sang à J7 et J24 pour l'administration de 10 µg de pXL3348. De plus, l'effet physiologique de l'augmentation d'érythropoiëtine qui se traduit par une augmentation de l'hématocrite est très important (85 %), dès J7 et ce, même pour une très faible quantité de plasmide (1 µg).

### Exemple 20 : transfert d'un gène codant pour le facteur de croissance de l'endothélium vasculaire (VEGF)

Des souris C57B16 ou SCID adultes ont reçu, dans le muscle tibial cranial et de manière bilatérale, une injection de pCOR hVEGF (pXL3212 , 15 µg).

Le plasmide pXL3212 (Figure 11) est un vecteur dérivé du plasmide pXL2774 (WO97/10343) dans lequel l'ADNc codant pour le VEGF165 (Vascular Endothelial Growth Factor, Genbank: HUMEGFAA) a été introduit sous contrôle du promoteur provenant de la région précoce du cytomegalovirus humain (hCMV IE) et du signal de poly-adénylation de la région tardive du virus SV40 (Genbank SV4CG).

Les conditions d'électrotransfert sont les suivantes : intensité champ électrique 250 V/cm, 8 impulsions de 20 msec., fréquence 2 Hz. Les prélèvements sanguins ont été réalisés au niveau du plexus rétroorbitaire. Les prélèvements ont été effectués un jour avant, et sept jours après, l'injection du plasmide. Le dosage immuno-enzymatique du VEGF humain a été réalisé à l'aide du kit Quantikine (R&D System). Le test a été étalonné avec du VEGF humain dans du sérum de souris. Les résultats sont présentés dans le tableau 14

**Tableau 14 :**

| concentration sérique (ng/litre) en VEGF chez des souris C57B16 et SCID. | | | |
|---|---|---|---|
| Lignée de souris | Jour du dosage | Electrotransfert | VEGF humain (ng/litre) |
| C57BL6 | J -1 | - | non détectable |
| C57BL6 | J +7 | + | 393 ± 110 |
| SCID | J -1 | - | non détectable |
| SCID | J +7 | + | 99 ± 26 |

### Exemple 21 : transfert d'un gène codant pour le facteur IX.

Des souris C57B16 ou SCID adultes ont reçu, dans le muscle tibial cranial et de manière bilatérale, une injection de pXL3388 (15 µg).

Le plasmide pXL3388 (Figure 12) est un vecteur dérivé du plasmide pXL2774 (WO97/10343) dans lequel l'ADNc codant pour le facteur IX humain (facteur Christmas), Genbank: HUMFIXA) a été introduit sous contrôle du promoteur provenant de la région précoce du cytomegalovirus humain (hCMV IE, Genbank HS5IEE) et du signal de poly-adénylation de la région tardive du virus SV40 (Genbank SV4CG).

Les conditions d'électrotransfert sont les suivantes : intensité champ électrique 200 V/cm, 8 impulsions de 20 msec., fréquence 2 Hz. Les prélèvements sanguins ont été réalisés au niveau du plexus rétroorbitaire. Les prélèvements ont été effectués sept jours après injection du plasmide. Les résultats sont présentés dans le tableau 15.

**Tableau 15 :**

| concentration plasmatique en facteur IX chez des souris C57B16 et SCID. | | | |
|---|---|---|---|
| Lignée de souris | injection | Electrotransfert | Facteur IX humain (µg/L) |
| C57BL/6 | pXL3388 | + | 69 ± 12 |
| C57BL/6 | pXL3388 | - | non détectable |
| C57BL/6 | NaCl 0,9 % | + | non détectable |
| SCID | pXL3388 | + | 66 ± 5 |
| SCID | pXL3388 | - | non détectable |

Le facteur IX humain n'est détectable dans le sang que lorsque le plasmide a été administré dans les conditions du procédé selon l'invention.

### Exemple 22 : transfert d'un gène codant pour le facteur de croissance des fibroblastes 1 (FGF1).

Des souris C57BL6 ou SCID adultes ont reçu, dans le muscle tibial cranial et de manière bilatérale, une injection de pCOR FGF1 (pXL3096, 15 µg).

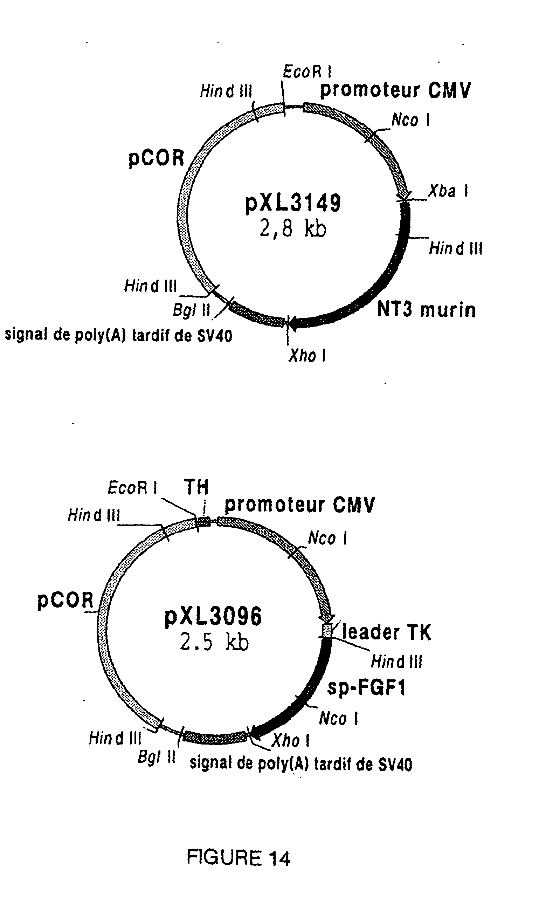

Le plasmide pXL3096 (Figure 14) est un vecteur dérivé du plasmide pXL2774 (WO97/10343) additionné d'une séquence capable de former une triple hélice (TH, Wils et al., 1997.Gene Ther 4:323-330) dans lequel le gène codant pour une fusion entre le peptide signal de l'interféron de fibroblastes humain et l'ADNc du FGF1 (Fibroblast Growth Factor1) (sp-FGF1, Jouanneau et al., 1991 PNAS 88:2893-2897) a été introduit sous contrôle du promoteur provenant de la région précoce du cytomegalovirus humain (hCMV IE) suivi de la séquence leader (transcrite, non traduite) du gène TK de HSV1 et du signal de poly-adénylation de la région tardive du virus SV40 (Genbank SV4CG).

Les conditions d'électrotransfert sont les suivantes : intensité champ électrique 200 V/cm, 8 impulsions de 20 msec., fréquence 2 Hz. La présence de FGF1 est ensuite révélée par immuno-histochimie.

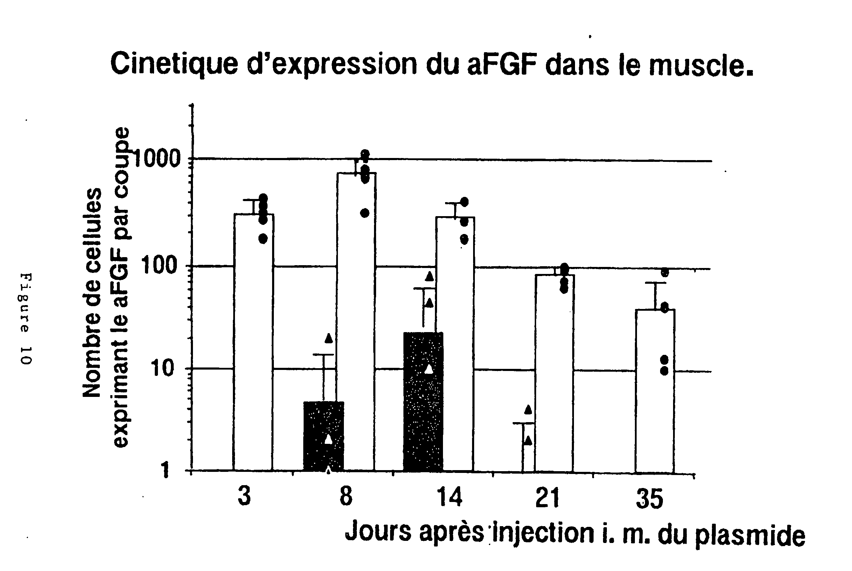

Les résultats pour les souris C57BL6 sont présentés sur la figure 10. On constate que le nombre de fibres positives est très largement supérieur pour le groupe soumis au champ électrique par rapport au groupe contrôle (ayant reçu une injection de pXL3096 mais non soumis au champ électrique). La présence de FGF1 pour le groupe contrôle est quasiment indétectable à J21 et J35 alors qu'un nombre important de fibres positives reste observable pour les groupes traités par électrotransfert.

Les résultats pour les souris SCID sont présentés dans le tableau 16.

**Tableau 16 :**

| expression de FGF, étude immunohistochimique et nombre de fibres positives sur une section musculaire prise dans la partie médiane du muscle | | | |
|---|---|---|---|
| | Electrotransfert | Tibial cranial gauche | Tibial cranial droit |
| pXL 3096 (15µg) | + | 600 | 450 |
| | + | 700 | 300 |
| | | | |
| pXL 3096 (15µg) | - | 3 | 0 |
| | - | 3 | 0 |
| | - | 0 | 0 |
| | | | |
| pXL 3096 (1,5µg) | + | 80 | 70 |
| | + | 20 | 35 |
| | + | 110 | 100 |
| | | | |
| pXL 3096 (1,5µg) | - | 0 | 0 |
| | - | 0 | 1 |

L'expression de FGF1, telle que déterminée par le nombre de fibres positives révélées par immuno-histochimie, est détectée uniquement dans les muscles soumis au champ électrique. Il est à noter que l'expression de FGF1 est détectée même pour une faible dose de plasmide administré (1,5 µg).

### Exemple 23 : transfert d'un gène codant pour le facteur neurotrophique NT3

Le procédé selon l'invention a été appliqué sur la souris adulte (C57B16) et le souriceau *Xt*/*pmn* pour le transfert du gène codant pour la neurotrophine 3 (NT3). Les souris *pmn* constituent un modèle murin de la sclérose amyotrophique latérale (SLA) caractérisé par une dégénerescence précoce et rapide des motoneurones et par une durée de vie moyenne de 40 jours environ.

### 23. 1- transfert du gène codant pour NT3 dans des souris adulte

Des souris C57B1/6 âgées de 5 semaines ont reçu, dans le muscle tibial cranial et de manière unilatérale, une injection de plasmide pXL3149 (12,5 µg) comprenant le gène codant pour la neurotrophine 3 (NT-3) murine.

Le plasmide pXL3149 (Figure 14) est un vecteur dérivé du plasmide pXL2774 (WO97/10343) dans lequel le gène codant pour la neurotrophine 3 (NT-3) murine (Genbank MMNT3) a été introduit sous contrôle du promoteur provenant de la région précoce du cytomegalovirus humain (hCMV IE) et du signal de poly-adénylation de la région tardive du virus SV40 (Genbank SV4CG).

Les conditions d'électrotransfert sont les suivantes : intensité champ électrique 250 V/cm, 4 impulsions de 20 msec., fréquence 1 Hz. Le champ électrique est appliqué immédiatement après injection de l'ADN plasmidique. La présence de NT3 est recherchée dans le surnageant 12000 g des broyats musculaires en tampon PBS, 7 jours après le traitement des souris. La quantité de NT3 est mesurée par un dosage ELISA [Kit Promega].

Les valeurs moyennes (± intervalle de confiance 95 %) sur 20 muscles sont de 77 +/- 11 pg/muscle (ADN plasmidique administré sans électrotransfert) et de 2700 +/- 900 pg/muscle (ADN plasmidique administré avec électrotransfert).

On observe ainsi une augmentation d'un facteur 55 de la quantité de NT3 produite dans le muscle lorsque le plasmide pXL3149 est transféré dans les conditions du procédé selon l'invention.

### 23. 2- transfert du gène codant pour NT3 dans des souriceaux

Une expérience comparable a été réalisée sur des souris Xt pmn hétérozygotes agées de 4 à 5 jours avec le plasmide pXL3149. Les doses injectées sont 130 µg par animal et les injections sont réalisées en multisite dans différents muscles de l'animal (gastrocnémien 25 µg, tibial crantal 12,5 µg).

Les conditions d'électrotransfert sont les suivantes : intensité champ électrique 500 V/cm, 4 impulsions de 20 msec., fréquence 1 Hz.

La présence de NT3 est recherchée 7 jours après administration du plasmide dans le plasma et dans le muscle (gastrocnémien ou tibial cranial). Un témoin du niveau basal de NT3 est réalisé par administration d'une solution de NaCl 0,9 %. La quantité de NT3 est déterminée par un dosage ELISA [Kit Promega]. Les résultats sont présentés dans le tableau 17.

**Tableau 17 :**

| valeurs moyennes ± SEM de la quantité de NT3 (pg par muscle et pg par ml de plasma). | | | | |
|---|---|---|---|---|
| | NaCl 0,9 % | | pXL3149 | |
| Electrotransfert | - | + | - | + |
| Plasma | 0 (n=2) | 0 (n=2) | 46 ± 10 (n=4) | 1599 ± 639 (n=4) |
| Muscle gastrocnémien | 3619 ± 102 (n=4) | 1619 ± 150 (n=2) | 3647 ± 1078 (n=8) | 19 754 ± 3818 (n=8) |
| Muscle tibial cranial | 1415 ± 363 (n=4) | 1453 ± 375 (n=2) | 1400 ± 155 (n=8) | 16 826 ± 3135 (n=8) |

Dans les conditions de l'expérience, on observe un niveau basal du signal de détection de NT3 dans le muscle gastrocnémien et dans le muscle tibial cranial. En l'absence d'électrotransfert, le niveau d'expression du gène NT3 obtenu pour l'injection du plasmide pXL3149 n'est pas supérieur au niveau basal de détection de NT3 dans le muscle. Lorsque le plasmide est administré avec le procédé selon l'invention, on constate que la quantité de NT3 détectée dans le muscle est très significativement augmentée. On observe également que la quantité de NT3 sécrétée par le muscle et détectée dans le plasma est très nettement augmentée dans ces conditions (facteur augmentation - x 35).

Ces résultats démontrent, que pour une quantité d'ADN donnée, le procédé selon l'invention permet d'augmenter le façon très significative l'efficacité du transfert d'ADN et d'obtenir, non seulement dans le muscle mais également dans le plasma, une augmentation importante la quantité d'une neurothrophine telle que NT3.

### Exemple 24 : transfert du gène codant pour l'hormone de croissance humaine.

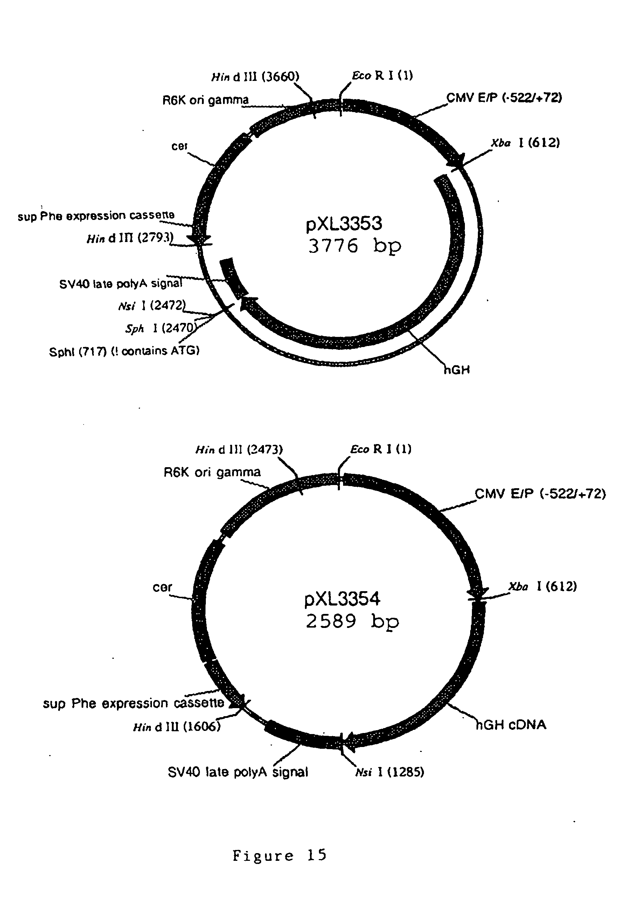

Des souris C57B1/6 ont reçu, dans le muscle tibial cranial et de manière unilatérale, une injection de plasmide pXL3353 (10 µg) ou de plasmide pXL3354 (10 µg). Le plasmide pXL3353 (figure 15) est un vecteur dérivé du plasmide pXL2774 dans lequel le gène entier de l'hormone de croissance humaine (fragment XbaI/SphI hGH qui s'étend du signal de début de transcription, site BamH1 , jusqu'à 224 bp après le site poly A) a été introduit sous contrôle du promoteur provenant de la région précoce du cytomegalovirus humain (hCMV IE) et du signal de poly-adénylation de la région tardive du virus SV40.

Le cDNA du gène de l'hormone de croissance humaine a été obtenu par transcription réverse d'une banque de mRNA poly(A+) de la glande pituitaire humaine suivie de 30 cycles d'amplification PCR avec les oligonucléotides suivants :
Oligonucléotide complémentaire de la région 5':
5'- GGGTCTAGAGCCACCATGGCTACAGGCTCCCGGAC -3'

Cet oligonucléotide contient un site XbaI et la séquence kozak.
Oligonucléotide complémentaire de la région 3' :
5'- GGGATGCATTTACTAGAAGCCACAGCTGCCTC-3'

Cet oligonucléotide contient un site NsiI et le codon stop.

Le fragment amplifié a été introduit dans le plasmide pCR2.1 (TA Cloning kit, Invitrogen) et séquencé. Un fragment XbaI/NsiI de 681 bp contenant le cDNA de hGH a été ligaturé avec le fragment XbaI/Nsi1 de pXL3353 pour générer le plasmide pXL3354 (figure 15).

Les conditions d'électrotransfert sont les suivantes : intensité champ électrique 200 V/cm, 8 impulsions de 20 msec., fréquence 1 Hz. Le champ électrique est appliqué immédiatement après injection de l'ADN plasmidique. La présence de hGH est recherchée, 7 jours après le traitement des souris, dans le surnageant des broyats musculaires en tampon PBS centrifugés à 12 000 g. La quantité de hGH est mesurée par un dosage ELISA (Boehringer Manheim).

**Tableau18 :**

| valeurs moyennes ± SEM de la protéine hGH (picogrammes)/muscle | | | | |
|---|---|---|---|---|
| | Injection hGH génomique (pXL3353) | | Injection hGH cDNA (pXL3354) | |
| Electrotransfert | - | + | - | + |
| Muscle Tibial Cranial | 87,1 ± 9,3 (n=10) | 1477,6 ± 67,6 (n=10) | 2820,0 ± 487,5 (n=10) | 15739,1 ± 915,5 (n=10) |

Ces résultats montrent que l'électrotransfert permet d'obtenir une augmentation très importante l'hormone de croissance humaine. Il est à noter que cette amplification est également observée avec le plasmide contenant le gène entier avec toutes ses séquences de régulation.

### Exemple 25 : Effet de l'électrotransfert sur l'expression de transgènes vaccinaux

Cet exemple met en évidence que le procédé selon l'invention est également applicable au transfert de gènes codant pour des polypeptides d'intérêt vaccinal.

L'expérience est réalisée chez des souris Balb/c femelles agées de 9 semaines. Les électrodes utilisées sont des électrodes plates en acier inoxydable distantes de 5 mm. Le VR-HA est un ADN plasmidique comportant le gène de l'hémagglutinine du virus de la grippe (souche A/PR/8/34). Le VR-gB est un ADN plasmidique comportant le gène de la glycoprotéine B (gB) du cytomégalovirus humain (souche Towne).

La solution de plasmide (50 µl d'une solution à 20 µg /ml ou 200 µg /ml dans NaCl 0,9 %) est injectée longitudinalement à travers la peau dans le muscle tibial cranial de manière unilatérale. Les impulsions électriques sont appliquées 20 sec après l'administration du plasmide, perpendiculairement à l'axe du muscle à l'aide d'un générateur d'impulsions carrées ( intensité champ électrique 200 V/cm, 8 impulsions consécutives d'une durée de 20 msec, fréquence 1 Hz).

Pour l'évaluation de la stimulation de la réponse immunitaire, le protocole d'immunisation suivant a été suivi :
- J 0: prélévement du sérum préimmun
- J 1: primo-injection, plus ou moins électrotransfert
- J 2: prélèvement du sérum immun
- J 2: injection de rappel, plus ou moins électrotransfert
- J 42: prélèvement de sérum immun
- J 63: prélèvement de sérum immun

Les prélèvements sanguins sont effectués au niveau du sinus rétro-orbital. Les dosages des anticorps spécifiques sont réalisés par ELISA. Chaque condition expérimentale est testée sur 10 animaux injectés en unilatéral.

Les résultats concernant les titres en anticorps dirigés contre l'hémagglutinine du virus de la grippe sont présentés dans le tableau 19A.

**Tableau 19-a :**

| titres en anticorps dirigés contre l'hémagglutinine du virus de la grippe, obtenus après injection de 1 ou 10 µg d'ADN (VR-HA) en absence ou en présence d'impulsions électriques. Les résultats sont les moyennes géométriques de 10 animaux (8 animaux pour le groupe injecté avec 1 µg d'ADN en présence d'impulsions électriques et prélevés à J63) ± écart-type. La valeur de p a été obtenue par comparaison deux à deux des groupes injectés en présence et en absence d'impulsions électriques en utilisant le test non paramétrique de Mann-Whitney. | | | | | |
|---|---|---|---|---|---|
| | Electrotransfert | J0 | J21 | J42 | J63 |
| VR-HA (1 µg) | - | < 50 | 132 ± 739 | 1201 ± 4380 | 1314 ± 2481 |
| VR-HA (1 µg) (p) | + | < 50 | 1121 ± 1237 (0,0135) | 10441 ± 7819 (0,0022) | 8121 ± 5619 (0,0033) |
| VR-HA (10 µg) | - | <50 | 781 ± 666 | 5113 ± 16015 | 4673 ± 8238 |
| VR-HA (10 µg) (p) | + | < 50 | 4153 ± 2344 (0,0002) | 74761 ± 89228 (0,0005) | 41765 ± 52361 (0,0007) |

Ces résultats montrent que les titres en anticorps dirigés contre l'hémagglutinine du virus de la grippe sont augmentés d'un facteur 10 environ dans les groupes soumis aux impulsions électriques. Ainsi les souris ayant reçu 1 µg d'ADN en présence d'impulsions électriques présentent un titre moyen en anticorps légèrement supérieur à celui des souris ayant reçu 10 µg d'ADN en absence d'impulsion électrique.

Les résultats concernant les titres en anticorps dirigés contre la glycoprotéine B du cytomégalovirus humain sont présentés dans le tableau 19B.

**Tableau 19 B :**

| titres en anticorps dirigés contre la glycoprotéine B (gB) du cytomagalovirus humain obtenus après injection de 10 µg d'ADN (VR-gB) en absence ou en présence d'impulsions électriques. Les résultats sont les moyennes géométriques de·10 animaux (9 animaux pour le groupe injecté en présence d'impulsions électriques) ± écart-type. La valeur de p a été obtenue par comparaison deux à deux des groupes injectés en présence et en absence d'impulsions électriques en utilisant le test non paramétrique de Mann-Whitney. | | | | | |
|---|---|---|---|---|---|
| | Electrotransfert | J 0 | J 21 | J 42 | J 63 |
| VR-gB (10 µg) | - | < 50 | 73 ± 138 | 755 ± 1766 | 809 ± 1363 |
| VR-gB (10 µg) (p) | + | < 50 | 200 ± 119 (0,0558) | 3057 ± 1747 (0,0108) | 2112 ± 1330 (0,0479) |

Ces résultats montrent que les titres en anticorps dirigés contre la glycoprotéine B du cytomégalovirus humain sont augmentés d'un facteur 4 à J42, dans le groupe soumis aux impulsions électriques. On note également que le coefficient de variation est en moyenne trois fois plus faible dans les groupes d'animaux soumis aux impulsions électriques.

## Revendications

1. Utilisation d'acide nucléique pour la préparation d'une composition destinée à être transférée in vivo dans un ou plusieurs muscles striés, **caractérisée en ce que** ladite composition est mise en contact avec des cellules du muscle, cette mise en contact étant simultanée avec ou suivie de l'application audit muscle d'une ou plusieurs impulsions électriques d'ondes unipolaires d'une intensité comprise entre 4 et 400 volts/cm.

2. Utilisation selon la revendication 1, **caractérisée en ce que** ladite composition à transférer est destinée au traitement par thérapie génique.

3. Utilisation selon la revendication 2, **caractérisée en ce que** ladite composition est destinée à une application vaccinale ou immunostimulante.

4. Utilisation l'une des revendications 1 à 3, **caractérisée en ce que** la mise en contact est effectuée par administration directe dans le tissu ou par administration topique ou systémique.

5. Utilisation selon l'une des revendications 1 à 4, **caractérisée en ce que** les ondes sont des ondes unipolaires oscillantes de durée limitée.

6. Utilisation selon l'une des revendications 1 à 5, **caractérisée en ce que** l'intensité du champ est comprise entre 30 et 300 volts/cm.

7. Utilisation selon l'une des revendications 1 à 6, **caractérisée en ce que** la durée totale d'application du champ électrique est supérieure à 10 millisecondes.

8. Utilisation selon l'une des revendications 1 à 7, **caractérisée en ce que** l'application au muscle du champ électrique comprend une ou plusieurs impulsions de fréquence régulière.

9. Utilisation selon la revendication 8, **caractérisée en ce que** l'application au muscle du champ électrique comprend entre 1 et 100 000 impulsions de fréquence comprise entre 0,1 et 1000 hertz.

10. Utilisation selon l'une des revendications 1 à 7, **caractérisée en ce que** les impulsions électriques sont délivrées de façon irrégulière les unes par rapport aux autres et **en ce que** la fonction décrivant l'intensité du champ électrique en fonction du temps d'une impulsion est variable.

11. Utilisation selon l'une des revendications 1 à 10, **caractérisée en ce que** l'intégrale de la fonction décrivant la variation du champ électrique avec le temps est supérieure à 1 kVxmsec/cm.

12. Utilisation selon la revendication 11, **caractérisée en ce que** cette intégrale est supérieure ou égale à 5 kVxmsec/cm.

13. Utilisation selon l'une des revendications 1 à 12, **caractérisée en ce que** les impulsions électriques comprennent des impulsions à ondes carrées.

14. Utilisation selon l'une des revendications 1 à 13, **caractérisée en ce que** les impulsions électriques sont appliquées avec des électrodes placées de part et d'autre du muscle ou placées au contact de la peau.

15. Utilisation selon l'une des revendications 1 à 13, **caractérisée en ce que** les impulsions électriques sont appliquées avec des électrodes introduites à l'intérieur du muscle.

16. Utilisation selon l'une des revendications 1 à 15, **caractérisée en ce que** l'acide nucléique est injecté dans le muscle.

17. Utilisation selon l'une des revendications 1 à 15, **caractérisée en ce que** l'acide nucléique est injecté par voie systémique.

18. Utilisation selon la revendication 17, **caractérisée en ce que** l'acide nucléique est injecté par voie intra-artérielle ou intra-veineuse.

19. Utilisation selon l'une des revendications 1 à 15, **caractérisée en ce que** l'acide nucléique est administré par voie topique, cutanée, orale, vaginale, intranasale, sous cutanée ou intra-oculaire.

20. Utilisation selon l'une des revendications 1 à 19, **caractérisée en ce que** l'acide nucléique est présent dans une composition contenant, en outre, des excipients pharmaceutiquement acceptables pour les différents modes d'administration.

21. Utilisation selon la revendication 20, **caractérisée en ce que** la composition est adaptée à l'administration parentérale.

22. Utilisation selon l'une des revendications 1 à 21, **caractérisée en ce que** l'acide nucléique est un acide désoxyribonucléique.

23. Utilisation selon l'une des revendications 1 à 21, **caractérisée en ce que** l'acide nucléique est un acide ribonucléique.

24. Utilisation selon l'une des revendications 1 à 23, **caractérisée en ce que** l'acide nucléique est d'origine synthétique ou biosynthétique, ou extrait d'un virus ou d'un organisme procaryote ou eucaryote unicellulaire ou pluricellulaire.

25. Utilisation selon la revendication 24, **caractérisée en ce que** l'acide nucléique administré est associé à tout ou partie des composants de l'organisme d'origine et/ou du système de synthèse.

26. Utilisation selon l'une des revendications 1 à 25, **caractérisée en ce que** l'acide nucléique code pour un ARN ou une protéine d'intérêt.

27. Utilisation selon la revendication 26, **caractérisée en ce que** l'ARN est un ARN catalytique ou antisens.

28. Utilisation selon la revendication 26, **caractérisée en ce que** l'acide nucléique code pour une protéine choisie parmi les enzymes, les dérivés sanguins, les hormones, les lymphokines, les facteurs de croissance, les facteurs trophiques, les facteurs angiogéniques, les facteurs neurotrophiques, les facteurs de croissance osseuse, les facteurs hématopoïétiques, les facteurs de coagulation, les antigènes et les protéines impliquées dans le métabolisme des acides aminés, des lipides et des autres constituants essentiels de la cellule.

29. Utilisation selon la revendication 28, **caractérisée en ce que** l'acide nucléique code pour les facteurs angiogéniques VEGF et FGF, les facteurs neurotrophiques BDNF, CNTF, NGF, IGF, GMF, FGF1, NT3, NT5, la protéine Gax, l'insuline pour le traitement du diabète, l'hormone de croissance, une cytokine, l'α-lantitrypsine, la calcitonine, la leptine et les apolipoprotéines, les enzymes de biosynthèse des vitamines, des hormones et des neuromédiateurs.

30. Utilisation selon la revendication 26, **caractérisée en ce que** l'acide nucléique code pour un anticorps, un fragment variable d'anticorps simple chaîne (ScFv) ou tout autre fragment d'anticorps possédant des capacités de reconnaissance dans un but d'immunothérapie, ou code pour un récepteur soluble, pour un peptide agoniste ou antagoniste d'un récepteur ou d'une protéine d'adhésion, pour une protéine artificielle, chimérique ou tronquée.

31. Utilisation selon la revendication 30, **caractérisée en ce que** l'acide nucléique code pour un anticorps antiidiotype, un fragment soluble du récepteur CD4 ou du récepteur du TNFa ou du récepteur de l'acétylcholine.

32. Utilisation selon l'une des revendications 28 à 31, **caractérisée en ce que** l'acide nucléique code pour un précurseur d'une protéine thérapeutique.

33. Utilisation selon l'une des revendications 1 à 32, **caractérisée en ce que** l'acide nucléique est sous forme d'un plasmide.

34. Utilisation selon l'une des revendications 1 à 32, **caractérisée en ce que** l'acide nucléique contient un gène de grande taille et/ou des introns et/ou des éléments régulateurs de petite ou de grande taille.

35. Utilisation selon l'une des revendications 1 à 32, **caractérisée en ce que** l'acide nucléique est un ADN épisomal ou un chromosome artificiel de levure ou un minichromosome.

36. Utilisation selon l'une des revendications 1 à 35, **caractérisée en ce que** l'acide nucléique contient des séquences permettant et/ou favorisant l'expression du transgène dans le muscle.

37. Utilisation selon l'une des revendications 1 à 36, **caractérisée en ce que** l'acide est associé à tout type de vecteurs ou à toute combinaison de vecteurs permettant d'améliorer le transfert d'acide nucléique tels que des virus, des agents synthétiques ou biosynthétiques, ou encore des billes propulsées ou non.

38. Utilisation selon l'une des revendications 1 à 37, **caractérisée en ce que** le muscle est soumis à un traitement visant à améliorer le transfert de gène, un traitement de nature pharmacologique en application locale ou systémique, ou un traitement enzymatique, perméabilisant, chirurgical, mécanique, thermique ou physique.

39. Utilisation selon l'une des revendications 1 à 38, **caractérisée en ce qu'**elle permet de faire produire par le muscle un agent à des doses physiologiques et/ou thérapeutiques, soit dans les cellules musculaires, soit secrété.

40. Utilisation selon l'une des revendications 1 à 39, **caractérisée en ce qu'**elle permet de moduler la quantité de transgène exprimé en modulant le volume de tissu musculaire transfecté.

41. Utilisation selon la revendication 40, **caractérisée en ce qu'**elle permet de moduler le volume de tissu musculaire transfecté par l'utilisation de sites multiples d'administration.

42. Utilisation selon l'une des revendications 1 à 41, **caractérisée en ce qu'**elle permet de moduler la quantité de transgène exprimé en modulant le nombre, la forme, la surface et la disposition des électrodes, et en variant l'intensité, le nombre, la durée, la fréquence et la forme des impulsions, ainsi que la quantité et le volume d'administration de l'acide nucléique.

43. Utilisation selon l'une des revendications 1 à 42, **caractérisée en ce qu'**elle permet de contrôler la localisation des tissus transfectés par le volume de tissu soumis aux impulsions électriques locales.

44. Utilisation selon l'une des revendications 1 à 43, **caractérisée en ce qu'**elle permet un retour à la situation initiale par l'ablation de la zone de tissu transfectée.

45. Acide nucléique et champ électrique d'ondes unipolaires d'une intensité comprise entre 4 et 400 volts/cm, comme produit de combinaison pour leur administration séparée ou étalée dans le temps in vivo au muscle strié et pour la thérapie génique reposant sur l'électrotransfection *in vivo* dans le muscle strié, après administration de l'acide nucléique.

46. Produit de combinaison selon la revendication 45 **caractérisé en ce que** l'intensité du champ est comprise entre 30 et 300 volts/cm.

47. Produit de combinaison selon l'une des revendications 45 à 46, **caractérisé en ce que** la durée totale d'application du champ électrique est supérieure à 10 millisecondes.

48. Produit de combinaison selon l'une des revendications 45 à 47, **caractérisé en ce que** l'application au muscle du champ électrique comprend une ou plusieurs impulsions de fréquence régulière.

49. Produit de combinaison selon la revendication 48, **caractérisé en ce que** l'application au muscle du champ électrique comprend entre 1 et 100 000 impulsions de fréquence comprise entre 0,1 et 1000 hertz.

50. Produit de combinaison selon l'une des revendications 45 à 47, **caractérisé en ce que** les impulsions électriques sont délivrées de façon irrégulière les unes par rapport aux autres et **en ce que** la fonction décrivant l'intensité du champ électrique en fonction du temps d'une impulsion est variable.

51. Produit de combinaison selon l'une des revendications 45 à 50, **caractérisé en ce que** l'intégrale de la fonction décrivant la variation du champ électrique avec le temps est supérieure à 1 kVxmsec/cm.

52. Produit de combinaison selon la revendication 51, **caractérisé en ce que** cette intégrale est supérieure ou égale à 5 kVxmsec/cm.

53. Produit de combinaison selon l'une des revendications 45 à 52, **caractérisé en ce que** les impulsions électriques sont des ondes unipolaires oscillantes de durée limitée.

54. Produit de combinaison selon l'une des revendications 45 à 53, **caractérisé en ce que** les impulsions électriques comprennent des impulsions à ondes carrées.

55. Produit de combinaison selon l'une des revendications 45 à 54, **caractérisé en ce que** les impulsions électriques sont appliquées avec électrodes placées de part et d'autre du muscle ou placées au contact de la peau.

56. Produit de combinaison selon l'une des revendications 45 à 54, **caractérisé en ce que** les impulsions électriques sont appliquées avec des électrodes introduites à l'intérieur du muscle.

57. Produit de combinaison selon l'une des revendications 45 à 56, **caractérisé en ce que** l'acide nucléique est injecté dans le muscle.

58. Produit de combinaison selon l'une des revendications 45 à 56, **caractérisé en ce que** l'acide nucléique est injecté par voie systémique.

59. Produit de combinaison selon la revendication 58, **caractérisé en ce que** l'acide nucléique est injecté par voie intra-artérielle ou intra-veineuse.

60. Produit de combinaison selon l'une des revendications 45 à 56, **caractérisé en ce que** l'acide nucléique est administré par voie topique, cutanée, orale, vaginale, intranasale, sous cutanée ou intra-oculaire.

61. Produit de combinaison selon l'une des revendications 45 à 60, **caractérisé en ce que** l'acide nucléique est présent dans une composition contenant, en outre, des excipients pharmaceutiquement acceptables pour les différents modes d'administration.

62. Produit de combinaison selon la revendication 61, **caractérisé en ce que** la composition est adaptée à l'administration parentérale.

63. Produit de combinaison selon l'une des revendications 45 à 62, **caractérisé en ce que** l'acide nucléique est un acide désoxyribonucléique.

64. Produit de combinaison selon l'une des revendications 45 à 62, **caractérisé en ce que** l'acide nucléique est un acide ribonucléique.

65. Produit de combinaison selon l'une des revendications 45 à 64, **caractérisé en ce que** l'acide nucléique est d'origine synthétique ou biosynthétique, ou extrait d'un virus ou d'un organisme procaryote ou eucaryote unicellulaire ou pluricellulaire.

66. Produit de combinaison selon la revendication 65, **caractérisé en ce que** l'acide nucléique administré est associé à tout ou partie des composants de l'organisme d'origine et/ou du système de synthèse.

67. Produit de combinaison selon l'une des revendications 45 à 66, **caractérisé en ce que** l'acide nucléique code pour un ARN ou une protéine d'intérêt.

68. Produit de combinaison selon la revendication 67, **caractérisé en ce que** l'ARN est un ARN catalytique ou antisens.

69. Produit de combinaison selon la revendication 67, **caractérisé en ce que** l'acide nucléique code pour une protéine choisie parmi les enzymes, les dérivés sanguins, les hormones, les lymphokines, les cytokines, les facteurs de croissance, les facteurs trophiques, les facteurs angiogéniques, les facteurs neurotrophiques, les facteurs de croissance osseuse, les facteurs hématopoïétiques, les facteurs de coagulation, les antigènes et les protéines impliquées dans le métabolisme des acides aminés, des lipides et des autres constituants essentiels de la cellule.

70. Produit de combinaison selon la revendication 69, **caractérisé en ce que** l'acide nucléique code pour les facteurs angiogéniques VEGF et FGF, les facteurs neurotrophiques BDNF, CNTF, NGF, IGF, GMF, FGF1, NT3, NT5, la protéine Gax, l'insuline pour le traitement du diabète, l'hormone de croissance, l'α-1-antitrypsine, la calcitonine, la leptine et les apolipoprotéines, les enzymes de biosynthèse des vitamines, des hormones et des neuromédiateurs.

71. Produit de combinaison selon la revendication 67, **caractérisé en ce que** l'acide nucléique code pour un anticorps, un fragment variable d'anticorps simple chaîne (ScFv) ou tout autre fragment d'anticorps possédant des capacités de reconnaissance dans un but d'immunothérapie, ou code pour un récepteur soluble, pour un peptide agoniste ou antagoniste d'un récepteur ou d'une protéine d'adhésion, pour une protéine artificielle, chimérique ou tronquée.

72. Produit de combinaison selon la revendication 71, **caractérisé en ce que** l'acide nucléique code pour un anticorps antiidiotype, un fragment soluble du récepteur CD4 ou du récepteur du TNFa ou du récepteur de l'acétylcholine.

73. Produit de combinaison selon l'une des revendications 69 à 72, **caractérisé en ce que** l'acide nucléique code pour un précurseur d'une protéine thérapeutique.

74. Produit de combinaison selon l'une des revendications 45 à 73, **caractérisé en ce que** l'acide nucléique est sous forme d'un plasmide.

75. Produit de combinaison selon l'une des revendications 45 à 73, **caractérisé en ce que** l'acide nucléique contient un gène de grande taille et/ou des introns et/ou des éléments régulateurs de petite ou de grande taille.

76. Produit de combinaison selon l'une des revendications 45 à 73, **caractérisé en ce que** l'acide nucléique est un ADN épisomal ou un chromosome artificiel de levure ou bactérien ou un minichromosome.

77. Produit de combinaison selon l'une des revendications 45 à 76, **caractérisé en ce que** l'acide nucléique contient des séquences permettant et/ou favorisant l'expression du transgène dans le muscle.

78. Produit de combinaison selon l'une des revendications 45 à 77, **caractérisé en ce que** l'acide est associé à tout type de vecteurs ou à toute combinaison de vecteurs permettant d'améliorer le transfert d'acide nucléique tels que des virus, des agents synthétiques ou biosynthétiques, ou encore des billes propulsées ou non.

79. Produit de combinaison selon l'une des revendications 45 à 78, **caractérisé en ce que** le muscle est soumis à un traitement visant à améliorer le transfert de gène, un traitement de nature pharmacologique en application locale ou systémique, ou un traitement enzymatique, perméabilisant, chirurgical, mécanique, thermique ou physique.

80. Produit de combinaison selon l'une des revendications 45 à 79, **caractérisé en ce qu'**il permet de faire produire par le muscle un agent à des doses physiologiques et/ou thérapeutiques, soit dans les cellules musculaires, soit secrété.

81. Produit de combinaison selon l'une des revendications 45 à 79, **caractérisé en ce qu'**il permet de moduler la quantité de transgène exprimé en modulant le volume de tissu musculaire transfecté.

82. Produit de combinaison selon la revendication 81, **caractérisé en ce qu'**il permet de moduler le volume de tissu musculaire transfecté par l'utilisation de sites multiples d'administration.

83. Produit de combinaison selon l'une des revendications 45 à 82, **caractérisé en ce qu'**il permet de moduler la quantité de transgène exprimé en modulant le nombre, la forme, la surface et la disposition des électrodes, et en variant l'intensité de champ, le nombre, la durée, la fréquence et la forme des impulsions, ainsi que la quantité et le volume d'administration de l'acide nucléique.

84. Produit de combinaison selon l'une des revendications 45 à 83, **caractérisé en ce qu'**il permet de contrôler la localisation des tissus transfectés par le volume de tissu soumis aux impulsions électriques locales.

85. Produit de combinaison selon l'une des revendications 45 à 84, **caractérisé en ce qu'**il permet un retour à la situation initiale par l'ablation de la zone de tissu transfectée.

## Claims

1. Use of a nucleic acid for the preparation of a composition intended to be transferred in vivo into one or more striated muscles, **characterized in that** the said composition is brought into contact with cells of the muscle, wherein said contact is simultaneous with or followed by the application to the said muscle of one or more electrical pulses of unipolar waves of an intensity of between 4 and 400 volts/cm.

2. Use according to Claim 1, **characterized in that** the said composition to be transferred is intended for treatment by gene therapy.

3. Use according to Claim 2, **characterized in that** the said composition is intended for a vaccine or immunostimulant application.

4. Use of one of Claims 1 to 3, **characterized in that** the bringing into contact is carried out by direct administration to the tissue or by topical or systemic administration.

5. Use according to one of Claims 1 to 4,
**characterized in that** the waves are oscillating unipolar waves of limited duration

6. Use according to one of Claims 1 to 5, **characterized in that** the intensity of the field is between 30 and 300 volts/cm.

7. Use according to one of Claims 1 to 6, **characterized in that** the total duration of application of the electric field is greater than 10 milliseconds.

8. Use according to one of Claims 1 to 7, **characterized in that** the application, to the muscle, of the electric field comprises one or more pulses of regular frequency.

9. Use according to Claim 8, **characterized in that** the application, to the muscle, of the electric field comprises between 1 and 100,000 pulses of frequency between 0.1 and 1000 hertz.

10. Use according to one of Claims 1 to 7, **characterized in that** the electrical pulses are delivered in an irregular manner relative to each other and **in that** the function describing the intensity of the electric field as a function of time for one pulse is variable.

11. Use according to one of Claims 1 to 10, **characterized in that** the integral of the function describing the variation of the electric field with time is greater than 1 kVxmsec/cm.

12. Use according to Claim 11, **characterized in that** this integral is greater than or equal to 5 kVxmsec/cm.

13. Use according to one of Claims 1 to 12, **characterized in that** the electrical pulses comprise square wave pulses.

14. Use according to one of Claims 1 to 13, **characterized in that** the electrical pulses are applied with electrodes placed either side of the muscle or placed in contact with the skin.

15. Use according to one of Claims 1 to 13, **characterized in that** the electrical pulses are applied with electrodes introduced inside the muscle.

16. Use according to one of Claims 1 to 15, **characterized in that** the nucleic acid is injected into the muscle.

17. Use according to one of Claims 1 to 15, **characterized in that** the nucleic acid is injected by the systemic route.

18. Use according to Claim 17, **characterized in that** the nucleic acid is injected by the intra-arterial or intravenous route.

19. Use according to one of Claims 1 to 15, **characterized in that** the nucleic acid is administered by the topical, cutaneous, oral, vaginal, intranasal, subcutaneous or intraocular route.

20. Use according to one of Claims 1 to 19, **characterized in that** the nucleic acid is present in a composition containing, in addition, pharmaceutically acceptable excipients for the different modes of administration.

21. Use according to Claim 20, **characterized in that** the composition is suitable for parenteral administration.

22. Use according to one of Claims 1 to 21, **characterized in that** the nucleic acid is a deoxyribonucleic acid.

23. Use according to one of Claims 1 to 21, **characterized in that** the nucleic acid is a ribonucleic acid.

24. Use according to one of Claims 1 to 23, **characterized in that** the nucleic acid is of synthetic or biosynthetic origin, or extracted from a virus or from a unicellular or pluricellular eukaryotic or prokaryotic organism.

25. Use according to Claim 24, **characterized in that** the nucleic acid administered is combined with all or part of the components of the organism of origin and/or of the synthesis System.

26. Use according to one of Claims 1 to 25, **characterized in that** the nucleic acid encodes an RNA or a protein of interest.

27. Use according to Claim 26, **characterized in that** the RNA is a catalytic or antisense RNA.

28. Use according to Claim 26, **characterized in that** the nucleic acid encodes a protein chosen from enzymes, blood derivatives, hormones, lymphokines, growth factors, trophic factors, angiogenic factors, neurotrophic factors, bone growth factors, haematopoietic factors, coagulation factors, antigens and proteins involved in the metabolism of amino acids, lipids and other essential constituents of the cell.

29. Use according to Claim 28, **characterized in that** the nucleic acid encodes the angiogenic factors VEGF and FGF, the neurotrophic factors BDNF, CNTF,NGF, IGF, GMF, FGF1, NT3, NT5, the Gax protein, insulin for the treatment of diabetes, growth hormone, a cytokine, α-1-antitrypsin, calcitonin, leptin and the apolipoproteins, the enzymes for the biosynthesis of vitamins, hormones and neuromediators.

30. Use according to Claim 26, **characterized in that** the nucleic acid codes for an antibody, a variable fragment of single-chain antibody (ScFv) or any other antibody fragment possessing recognition capacities for the purposes of immunotherapy, or codes for a soluble receptor, a peptide which is an agonist or antagonist of a receptor or of an adhesion protein, for an artificial, chimeric or truncated protein.

31. Use according to Claim 30, **characterized in that** the nucleic acid encodes an antiidiotype antibody, a soluble fragment of the CD4 receptor or of the TNFa receptor or of the acetylcholine receptor.

32. Use according to one of Claims 28 to 31, **characterized in that** the nucleic acid encodes a precursor of a therapeutic protein.

33. Use according to one of Claims 1 to 32, **characterized in that** the nucleic acid is in the form of a plasmid.

34. Use according to one of Claims 1 to 32, **characterized in that** the nucleic acid contains a gene of large size and/or introns and/or regulatory elements of small or large size.

35. Use according to one of Claims 1 to 32, **characterized in that** the nucleic acid is an episomal DNA or a yeast artificial chromosome or a minichromosome.

36. Use according to one of Claims 1 to 35, **characterized in that** the nucleic acid contains sequences allowing and/or promoting the expression of the transgene in the muscle.

37. Use according to one of Claims 1 to 36, **characterized in that** the acid is combined with any type of vectors or with any combination of vectors which make it possible to improve the transfer of nucleic acid, such as viruses, synthetic or biosynthetic agents, or beads which are propelled or otherwise.

38. Use according to one of Claims 1 to 37, **characterized in that** the muscle is subjected to a treatment intended to improve gene transfer, a treatment of pharmacological nature in the form of a local or systemic application, or an enzymatic, permeabilizing, surgical, mechanical, thermal or physical treatment.

39. Use according to one of Claims 1 to 38, **characterized in that** it makes it possibleto cause the muscle to produce an agent at physiological and/or therapeutic doses, either in the muscle cells, or secreted.

40. Use according to one of Claims 1 to 39, **characterized in that** it makes it possible to modulate the quantity of transgene expressed by modulating the volume of muscle tissue transfected.

41. Use according to Claim 40, **characterized in that** it makes it possible to modulate the volume of muscle tissue transfected by the use of multiple sites of administration.

42. Use according to one of Claims 1 to 41, **characterized in that** it makes it possible to modulate the quantity of transgene expressed by modulating the number, shape, surface and arrangement of the electrodes, and by varying the intensity, the number, the duration, the frequency and the form of the pulses, as well as the quantity and the volume of nucleic acid for administration.

43. Use according to one of Claims 1 to 42, **characterized in that** it makes it possible to control the localization of the tissues transfected by the volume of tissue subjected to the local electrical pulses.

44. Use according to one of Claims 1 to 43, **characterized in that** it allows a return to the initial situation by removal of the transfected tissue area.

45. Nucleic acid and unipolar-wave electric field of an intensity between 4 and 400 volts/cm, as combination product for their administration simultaneously, separately or spaced out over time in vivo to the striated muscle and for gene therapy based on in vivo electrotransfection into the striated muscle, after administration of the nucleic acid.

46. Combination product according to Claim 45, **characterized in that** the field intensity is between 30 and 300 volts/cm.

47. Combination product according to one of Claims 45 to 46, **characterized in that** the total duration of application of the electric field is greater than 10 milliseconds.

48. Combination product according to one of Claims 45 to 47, **characterized in that** the application, to the muscle, of the electric field comprises one or more pulses of regular frequency.

49. Combination product according to Claim 48, **characterized in that** the application, to the muscle, of the electric field comprises between 1 and 100,000 pulses of frequency between 0.1 and 1000 hertz.

50. Combination product according to one of Claims 45 to 47, **characterized in that** the electrical pulses are delivered in an irregular manner relative to each other and **in that** the function describing the intensity of the electric field as a function of the time for one pulse is variable.

51. Combination product according to one of Claims 45 to 50, **characterized in that** the integral of the function describing the variation of the electric field with time is greater than 1 kVxmsec/cm.

52. Combination product according to Claim 51, **characterized in that** this integral is greater than or equal to 5 kVxmsec/cm.

53. Combination product according to one of Claims 45 to 52, **characterized in that** the electrical pulses are oscillating unipolar waves of limited duration.

54. Combination product according to one of Claims 45 to 53, **characterized in that** the electrical pulses comprise square wave pulses.

55. Combination product according to one of Claims 45 to 54, **characterized in that** the electrical pulses are applied with electrodes placed either side of the muscle or placed in contact with the skin.

56. Combination product according to one of Claims 45 to 54, **characterized in that** the electrical pulses are applied with electrodes introduced inside the muscle.

57. Combination product according to one of Claims 45 to 56, **characterized in that** the nucleic acid is injected into the muscle.

58. Combination product according to one of Claims 45 to 56, **characterized in that** the nucleic acid is injected by the systemic route.

59. Combination product according to Claim 58, **characterized in that** the nucleic acid is injected by the intra-arterial or intravenous route.

60. Combination product according to one of Claims 45 to 56, **characterized in that** the nucleic acid is administered by the topical, cutaneous, oral, vaginal, intranasal, subcutaneous or intraocular route.

61. Combination product according to one of Claims 45 to 60, **characterized in that** the nucleic acid is present in a composition containing, in addition, pharmaceutically acceptable excipients for the different modes of administration.

62. Combination product according to Claim 61, **characterized in that** the composition is suitable for parenteral administration.

63. Combination product according to one of Claims 45 to 62, **characterized in that** the nucleic acid is a deoxyribonucleic acid.

64. Combination product according to one of Claims 45 to 62, **characterized in that** the nucleic acid is a ribonucleic acid.

65. Combination product according to one of Claims 45 to 64, **characterized in that** the nucleic acid is of synthetic or biosynthetic origin, or extracted from a virus or a unicellular or pluricellular eukaryotic or prokaryotic organism.

66. Combination product according to Claim 65, **characterized in that** the nucleic acid administered is combined with all or part of the components of the organism of origin and/or of the synthesis system.

67. Combination product according to one of Claims 45 to 66, **characterized in that** the nucleic acid encodes an RNA or a protein of interest.

68. Combination product according to Claim 67, **characterized in that** the RNA is a catalytic or antisense KNA.

69. Combination product according to Claim 67, **characterized in that** the nucleic acid encodes a protein chosen from enzymes, blood derivatives, hormones, lymphokines, cytokines, growth factors, trophic factors, angiogenic factors, neurotrophic factors, bone growth factors, haematopoietic factors, coagulation factors, antigens and proteins involved in the metabolism of amino acids, lipids and other essential constituents of the cell.

70. Combination product according to Claim 69, **characterized in that** the nucleic acid encodes the angiogenic factors VEGF and FGF, the neurotrophic factors BDNF, CNTF, NGF, IGF, GMF, FGF1, NT3, NT5, the Gax protein, insulin for the treatment of diabetes, growth hormone, α-1-antitrypsin, calcitonin, leptin and the apolipoproteins, the enzymes for the biosynthesis of vitamins, hormones and neuromediators.

71. Combination product according to Claim 67, **characterized in that** the nucleic acid codes for an antibody, a variable fragment of single-chain antibody (ScFv) or any other antibody fragment possessing recognition capacities for the purposes of immunotherapy, or codes for a soluble receptor, a peptide which is an agonist or antagonist of a receptor or of an adhesion protein, for an artificial, chimeric or truncated protein.

72. Combination product according to Claim 71, **characterized in that** the nucleic acid encodes an antiidiotype antibody, a soluble fragment of the CD4 receptor or of the TNFa receptor or of the acetylcholine receptor.

73. Combination product according to one of Claims 69 to 72, **characterized in that** the nucleic acid encodes a precursor of a therapeutic protein.

74. Combination product according to one of Claims 45 to 73, **characterized in that** the nucleic acid is in the form of a plasmid.

75. Combination product according to one of Claims 45 to 73, **characterized in that** the nucleic acid contains a gene of large size and/or introns and/or regulatory elements of small or large size.

76. Combination product according to one of Claims 45 to 73, **characterized in that** the nucleic acid is an episomal DNA or a yeast or bacterial artificial chromosome or a minichromosome.

77. Combination product according to one of Claims 45 to 76, **characterized in that** the nucleic acid contains sequences allowing and/or promoting the expression of the transgene in the muscle.

78. Combination product according to one of Claims 45 to 77, **characterized in that** the acid is combined with any type of vectors or with any combination of vectors which make it possible to improve the transfer of nucleic acid, such as viruses, synthetic or biosynthetic agents, or beads which are propelled or otherwise.

79. Combination product according to one of Claims 45 to 78, **characterized in that** the muscle is subjected to a treatment intended to improve gene transfer, a treatment of pharmacological nature in the form of a local or systemic application, or an enzymatic, permeabilizing, surgical, mechanical, thermal or physical treatment.

80. Combination product according to one of Claims 45 to 79, **characterized in that** it makes it possible to cause the muscle to produce an agent at physiological and/or therapeutic doses, either in the muscle cells, or secreted.

81. Combination product according to one of Claims 45 to 79, **characterized in that** it makes it possible to modulate the quantity of transgene expressed by modulating the volume of muscle tissue transfected.

82. Combination product according to Claim 81, **characterized in that** it makes it possible to modulate the volume of muscle tissue transfected by the use of multiple sites of administration.

83. Combination product according to one of Claims 45 to 82, **characterized in that** it makes it possible to modulate the quantity of transgene expressed by modulating the number, shape, surface and arrangement of the electrodes, and by varying the field intensity, the number, the duration, the frequency and the form of the pulses, as well as the quantity and the volume of nucleic acid for administration.

84. Combination product according to one of Claims 45 to 83, **characterized in that** it makes it possible to control the localization of the tissues transfected by the volume of tissue subjected to the local electrical pulses.

85. Combination product according to one of Claims 45 to 84, **characterized in that** it allows a return to the initial situation by removal of the transfected tissue area.

## Patentansprüche

1. Verwendung von Nukleinsäure zur Herstellung einer Zusammensetzung, welche dazu bestimmt ist, *in vivo* in einen oder mehrere quergestreifte Muskeln transferiert zu werden, **dadurch gekennzeichnet, dass** die Zusammensetzung mit Zellen des Muskels in Kontakt gebracht wird durch gleichzeitiger oder nachfolgender Anwendung von einem oder mehreren elektrischen Impulsen von einpoligen Wellen mit einer Intensität zwischen 4 und 400 Volt/cm auf den Muskel.

2. Verwendung nach Anspruch 1, **dadurch gekennzeichnet, dass** die zu transferierende Zusammensetzung für die Behandlung durch Gentherapie bestimmt ist.

3. Verwendung nach Anspruch 2, **dadurch gekennzeichnet, dass** die Zusammensetzung für eine Impfanwendung oder immunstimulierende Anwendung bestimmt ist.

4. Verwendung nach einem der Ansprüche 1 bis 3, **dadurch gekennzeichnet, dass** das Inkontaktbringen durch direkte Verabreichung in das Gewebe oder durch topische oder systemische Verabreichung erfolgt.

5. Verwendung nach einem der Ansprüche 1 bis 4, **dadurch gekennzeichnet, dass** die Wellen einpolige oszillierende Wellen von begrenzter Dauer sind.

6. Verwendung nach einem der Ansprüche 1 bis 5, **dadurch gekennzeichnet, dass** die Intensität des Felds zwischen 30 und 300 Volt/cm liegt.

7. Verwendung nach einem der Ansprüche 1 bis 6, **dadurch gekennzeichnet, dass** die gesamte Dauer der Anwendung des elektrischen Felds über 10 Millisekunden beträgt.

8. Verwendung nach einem der Ansprüche 1 bis 7, **dadurch gekennzeichnet, dass** die Anwendung des elektrischen Felds auf den Muskel einen oder mehrere Impulse von regelmässiger Frequenz umfasst.

9. Verwendung nach Anspruch 8, **dadurch gekennzeichnet, dass** die Anwendung des elektrischen Felds auf den Muskel zwischen 1 und 100000 Impulse mit einer Frequenz zwischen 0,1 und 1000 Hertz umfasst.

10. Verwendung nach einem der Ansprüche 1 bis 7, **dadurch gekennzeichnet, dass** die elektrischen Impulse bezogen aufeinander auf unregelmässige Weise abgegeben werden und dass die Funktion, welche die Intensität des elektrischen Felds in Abhängigkeit von der Zeit eines Impulses beschreibt, variabel ist.

11. Verwendung nach einem der Ansprüche 1 bis 10, **dadurch gekennzeichnet, dass** das Integral der Funktion, welche die Variation des elektrischen Felds mit der Zeit beschreibt, über 1 kV x ms/cm beträgt.

12. Verwendung nach Anspruch 11, **dadurch gekennzeichnet, dass** dieses Integral über oder gleich 5 kV x ms/cm beträgt.

13. Verwendung nach einem der Ansprüche 1 bis 12, **dadurch gekennzeichnet, dass** die elektrischen Impulse Impulse mit Rechteckwellen umfassen.

14. Verwendung nach einem der Ansprüche 1 bis 13, **dadurch gekennzeichnet, dass** die elektrischen Impulse mittels Elektroden, die auf beiden Seiten des Muskels angeordnet sind oder in Kontakt mit der Haut angeordnet sind, angewendet werden.

15. Verwendung nach einem der Ansprüche 1 bis 13, **dadurch gekennzeichnet, dass** die elektrischen Impulse mittels Elektroden, die in das Innere des Muskels eingeführt worden sind, angewendet werden.

16. Verwendung nach einem der Ansprüche 1 bis 15, **dadurch gekennzeichnet, dass** die Nukleinsäure in den Muskel injiziert wird.

17. Verwendung nach einem der Ansprüche 1 bis 15, **dadurch gekennzeichnet, dass** die Nukleinsäure systemisch injiziert wird.

18. Verwendung nach Anspruch 17, **dadurch gekennzeichnet, dass** die Nukleinsäure intra-arteriell oder intravenös injiziert wird.

19. Verwendung nach einem der Ansprüche 1 bis 15, **dadurch gekennzeichnet, dass** die Nukleinsäure auf topischem, kutanem, oralem, vaginalem, intranasalem, subkutanem oder intraokularem Wege verabreicht wird.

20. Verwendung nach einem der Ansprüche 1 bis 19, **dadurch gekennzeichnet, dass** die Nukleinsaure in einer Zusammensetzung vorliegt, welche ausserdem pharmazeutisch annehmbare Träger oder Vehikel für die verschiedenen Verabreichungsweisen enthält.

21. Verwendung nach Anspruch 20, **dadurch gekennzeichnet, dass** die Zusammensetzung für die parenterale Verabreichung angepasst ist.

22. Verwendung nach einem der Ansprüche 1 bis 21, **dadurch gekennzeichnet, dass** die Nukleinsäure eine Desoxyribonukleinsäure ist.

23. Verwendung nach einem der Ansprüche 1 bis 21, **dadurch gekennzeichnet, dass** die Nukleinsäure eine Ribonukleinsäure ist.

24. Verwendung nach einem der Ansprüche 1 bis 23, **dadurch gekennzeichnet, dass** die Nukleinsäure von synthetischem oder biosynthetischem Ursprung ist oder aus einem Virus oder einem prokaryotischen oder eukaryotischen einzelligen oder mehrzelligen Organismus extrahiert wird.

25. Verwendung nach Anspruch 24, **dadurch gekennzeichnet, dass** die verabreichte Nukleinsäure mit der Gesamtheit oder einem Teil der Bestandteile des Herkunftsorganismus und/oder des Synthesesystems assoziiert ist.

26. Verwendung nach einem der Ansprüche 1 bis 25, **dadurch gekennzeichnet, dass** die Nukleinsäure eine RNA oder ein Protein von Interesse kodiert.

27. Verwendung nach Anspruch 26, **dadurch gekennzeichnet, dass** die RNA eine katalytische RNA oder Antisinn-RNA ist.

28. Verwendung nach Anspruch 26, **dadurch gekennzeichnet, dass** die Nukleinsäure ein Protein kodiert, welches unter den Enzymen, den Blutderivaten, den Hormonen, den Lymphokinen, den Wachstumsfaktoren, den trophischen Faktoren, den angiogenetischen Faktoren, den neurotrophen Faktoren, den Knochenwachstumsfaktoren, den hämopoetischen Faktoren, den Gerinnungsfaktoren, den Antigenen und den Proteinen, die an dem Stoffwechsel der Aminosäuren, der Lipide und der anderen essentiellen Bestandteile der Zelle beteiligt sind, ausgewählt wird.

29. Verwendung nach Anspruch 28, **dadurch gekennzeichnet, dass** die Nukleinsäure die angiogenetischen Faktoren VEGF und FGF, die neurotrophen Faktoren BDNF, CNTF, NGF, IGF, GMF, FGF1, NT3, NT5, das Protein Gax, Insulin für die Behandlung von Diabetes, Wachstumshormon, ein Zytokin, α-1-Antitrypsin, Calcitonin, Leptin und die Apolipoproteine, die Enzyme der Biosynthese der Vitamine, Hormone und Neuromediatoren kodiert.

30. Verwendung nach Anspruch 26, **dadurch gekennzeichnet, dass** die Nukleinsäure einen Antikörper, ein variables Antikörper-Einzelkettenfragment (ScFv) oder ein jegliches anderes Antikörperfragment, welches Erkennungsfähigkeiten aufweist, mit einem Ziel einer Immuntherapie kodiert oder einen löslichen Rezeptor, ein Agonisten- oder Antagonisten-Peptid eines Rezeptors oder eines Adhäsionsproteins, ein künstliches, chimäres oder verkürztes Protein kodiert.

31. Verwendung nach Anspruch 30, **dadurch gekennzeichnet, dass** die Nukleinsäure einen Anti-Idiotyp-Antikörper, ein lösliches Fragment des CD4-Rezeptors oder des TNFa-Rezeptors oder des Acetylcholinrezeptors kodiert.

32. Verwendung nach einem der Ansprüche 28 bis 31, **dadurch gekennzeichnet, dass** die Nukleinsäure eine Vorstufe eines therapeutischen Proteins kodiert.

33. Verwendung nach einem der Ansprüche 1 bis 32, **dadurch gekennzeichnet, dass** die Nukleinsäure in Form eines Plasmids vorliegt.

34. Verwendung nach einem der Ansprüche 1 bis 32, **dadurch gekennzeichnet, dass** die Nukleinsäure ein Gen von grosser Grösse und/oder Introns und/oder regulatorische Elemente von kleiner oder von grosser Grösse enthält.

35. Verwendung nach einem der Ansprüche 1 bis 32, **dadurch gekennzeichnet, dass** die Nukleinsäure eine episomale DNA oder ein künstliches Hefe-Chromosom oder ein Minichromosom ist.

36. Verwendung nach einem der Ansprüche 1 bis 35, **dadurch gekennzeichnet, dass** die Nukleinsäure Sequenzen enthält, welche die Expression des Transgens in dem Muskel erlauben und/oder begünstigen.

37. Verwendung nach einem der Ansprüche 1 bis 36, **dadurch gekennzeichnet, dass** die Säure mit einer jeglichen Art von Vektoren oder einer jeglichen Kombination von Vektoren, welche erlauben, den Transfer von Nukleinsäure zu verbessern, wie Viren, synthetischen oder biosynthetischen Agentien oder ferner angetriebenen oder nicht-angetriebenen Kügelchen assoziiert ist

38. Verwendung nach einem der Ansprüche 1 bis 37, **dadurch gekennzeichnet, dass** der Muskel einer Behandlung, welche darauf abzielt, den Gentransfer zu verbessern, einer Behandlung von pharmakologischer Natur mit lokaler oder systemischer Anwendung oder einer enzymatischen, permeabilisierenden, chirurgischen, mechanischen, thermischen oder physikalischen Behandlung unterzogen wird.

39. Verwendung nach einem der Ansprüche 1 bis 38, **dadurch gekennzeichnet, dass** sie erlaubt, durch den Muskel ein Mittel in physiologischen und/oder therapeutischen Dosen, entweder in den Muskelzellen oder welches sekretiert wird, produzieren zu lassen.

40. Verwendung nach einem der Ansprüche 1 bis 39, **dadurch gekennzeichnet, dass** sie erlaubt, die Menge von exprimiertem Transgen zu modulieren, indem das Volumen von transfiziertem Muskelgewebe moduliert wird.

41. Verwendung nach Anspruch 40, **dadurch gekennzeichnet, dass** sie erlaubt, das Volumen von transfiziertem Muskelgewebe durch die Verwendung einer Mehrzahl von Verabreichungsstellen zu modulieren.

42. Verwendung nach einem der Ansprüche 1 bis 41, **dadurch gekennzeichnet, dass** sie erlaubt, die Menge von exprimiertem Transgen zu modulieren, indem die Anzahl, die Form, die Oberfläche und die Anordnung der Elektroden moduliert werden und indem die Intensität, die Anzahl, die Dauer, die Frequenz und die Form der Impulse sowie die Verabreichungsmenge und das Verabreichungsvolumen der Nukleinsäure variiert werden.

43. Verwendung nach einem der Ansprüche 1 bis 42, **dadurch gekennzeichnet, dass** sie erlaubt, die Lage der transfizierten Gewebe durch das Volumen von Gewebe, welches den örtlichen elektrischen Impulsen unterworfen wird, zu kontrollieren.

44. Verwendung nach einem der Ansprüche 1 bis 43, **dadurch gekennzeichnet, dass** sie eine Rückkehr zu der Ausgangssituation durch operative Entfernung der Zone von transfiziertem Gewebe erlaubt.

45. Nukleinsäure und elektrisches Feld von einpoligen Wellen mit einer Intensität zwischen 4 und 400 Volt/cm als Kombinationsprodukt für deren getrennte oder zeitlich gestaffelte Verabreichung *in vivo* an den quergestreiften Muskel und für die Gentherapie, beruhend auf einer Elektrotransfektion *in vivo* in den quergestreiften Muskel nach Verabreichung der Nukleinsäure.

46. Kombinationsprodukt nach Anspruch 45, **dadurch gekennzeichnet**, das die Intensität des Felds zwischen 30 und 300 Volt/cm beträgt.

47. Kombinationsprodukt nach einem der Ansprüche 45 bis 46, **dadurch gekennzeichnet, dass** die gesamte Dauer der Anwendung des elektrischen Felds über 10 Millisekunden beträgt.

48. Kombinationsprodukt nach einem der Ansprüche 45 bis 47, **dadurch gekennzeichnet, dass** die Anwendung des elektrischen Felds auf den Muskel einen oder mehrere Impulse von regelmässiger Frequenz umfasst.

49. Kombinationsprodukt nach Anspruch 48, **dadurch gekennzeichnet, dass** die Anwendung des elektrischen Felds auf den Muskel zwischen 1 und 100000 Impulse mit einer Frequenz zwischen 0,1 und 1000 Hertz umfasst.

50. Kombinationsprodukt nach einem der Ansprüche 45 bis 47, **dadurch gekennzeichnet, dass** die elektrischen Impulse bezogen aufeinander auf unregelmässig Weise abgegeben werden und dass die Funktion, welche die Intensität des elektrischen Felds in Abhängigkeit von der Zeit eines Impulses beschreibt, variabel ist.

51. Kombinationsprodukt nach einem der Ansprüche 45 bis 50, **dadurch gekennzeichnet, dass** das Integral der Funktion, welche die Variation des elektrischen Felds mit der Zeit beschreibt, über 1 kV x ms/cm beträgt.

52. Kombinationsprodukt nach Anspruch 51, **dadurch gekennzeichnet, dass** dieses Integral mehr als oder gleich 5 kV x ms/cm beträgt.

53. Kombinationsprodukt nach einem der Ansprüche 45 bis 52, **dadurch gekennzeichnet, dass** die elektrischen Impulse einpolige oszillierende Wellen von begrenzter Dauer sind.

54. Kombinationsprodukt nach einem der Ansprüche 45 bis 53, **dadurch gekennzeichnet, dass** die elektrischen Impulse Impulse mit Rechteckwellen umfassen.

55. Kombinationsprodukt nach einem der Ansprüche 45 bis 54, **dadurch gekennzeichnet, dass** die elektrischen Impulse mittels Elektroden, welche auf beiden Seiten des Muskels angeordnet sind oder in Kontakt mit der Haut angeordnet sind, angewendet werden.

56. Kombinationsprodukt nach einem der Ansprüche 45 bis 54, **dadurch gekennzeichnet, dass** die elektrischen Impulse mittels Elektroden, die in das Innere des Muskels eingeführt worden sind, angewendet werden.

57. Kombinationsprodukt nach einem der Ansprüche 45 bis 56, **dadurch gekennzeichnet, dass** die Nukleinsäure in den Muskel injiziert wird.

58. Kombinationsprodukt nach einem der Ansprüche 45 bis 56, **dadurch gekennzeichnet, dass** die Nukleinsäure systemisch injiziert wird.

59. Kombinationsprodukt nach Anspruch 58, **dadurch gekennzeichnet, dass** die Nukleinsäure intra-arteriell oder intravenös injiziert wird.

60. Kombinationsprodukt nach einem der Ansprüche 45 bis 56, **dadurch gekennzeichnet, dass** die Nukleinsäure auf topischem, kutanem, oralem, vaginalem, intranasalem, subkutanem oder intraokularem Wege verabreicht wird.

61. Kombinationsprodukt nach einem der Ansprüche 45 bis 60, **dadurch gekennzeichnet, dass** die Nukleinsäure in einer Zusammensetzung vorliegt, welche ausserdem pharmazeutisch annehmbare Träger oder Vehikel für die verschiedenen Verabreichungsweisen umfasst.

62. Kombinationsprodukt nach Anspruch 61, **dadurch gekennzeichnet, dass** die Zusammensetzung für eine parenterale Verabreichung angepasst ist.

63. Kombinationsprodukt nach einem der Ansprüche 45 bis 62, **dadurch gekennzeichnet, dass** die Nukleinsäure eine Desoxyribonukleinsäure ist.

64. Kombinationsprodukt nach einem der Ansprüche 45 bis 62, **dadurch gekennzeichnet, dass** die Nukleinsäure eine Ribonukleinsäure ist.

65. Kombinationsprodukt nach einem der Ansprüche 45 bis 64, **dadurch gekennzeichnet, dass** die Nukleinsäure von synthetischem oder biosynthetischem Ursprung ist oder aus einem Virus oder einem prokaryotischen oder eukaryotischen einzelligen oder mehrzelligen Organismus extrahiert wird.

66. Kombinationsprodukt nach Anspruch 65, **dadurch gekennzeichnet, dass** die verabreichte Nukleinsäure mit der Gesamtheit oder einem Teil der Bestandteile des Herkunftsorganismus und/oder des Synthesesystems assoziiert ist.

67. Kombinationsprodukt nach einem der Ansprüche 45 bis 66, **dadurch gekennzeichnet, dass** die Nukleinsäure eine RNA oder ein Protein von Interesse kodiert.

68. Kombinationsprodukt nach Anspruch 67, **dadurch gekennzeichnet, dass** die RNA eine katalytische RNA oder Antisinn-RNA ist.

69. Kombinationsprodukt nach Anspruch 67, **dadurch gekennzeichnet, dass** die Nukleinsäure ein Protein kodiert, welches unter den Enzymen, den Blutderivaten, den Hormonen, den Lymphokinen, den Zytokinen, den Wachstumsfaktoren, den trophischen Faktoren, den angiogenetischen Faktoren, den neurotrophen Faktoren, den Knochenwachstumsfaktoren, den hämopoetischen Faktoren, den Gerinnungsfaktoren, den Antigenen und den Proteinen, die an dem Stoffwechsel der Aminosäuren, der Lipide und der anderen essentiellen Bestandteile der Zelle beteiligt sind, ausgewählt wird.

70. Kombinationsprodukt nach Anspruch 69, **dadurch gekennzeichnet, dass** die Nukleinsäure die angiogenetischen Faktoren VEGF und FGF, die neurotrophen Faktoren BDNF, CNTF, NGF, IGF, GMF, FGF1, NT3, NT5, das Protein Gax, Insulin für die Behandlung von Diabetes, Wachstumshormon, α-1-Antitrypsin, Calcitonin, Leptin und die Apolipoproteine, die Enzyme der Biosynthese der Vitamine, Hormone und Neuromediatoren kodiert.

71. Kombinationsprodukt nach Anspruch 67, **dadurch gekennzeichnet, dass** die Nukleinsäure einen Antikörper, ein variables Antikörpereinzelkettenfragment (ScFv) oder ein jegliches anderes Antikörperfragment, welches Erkennungsfähigkeiten aufweist, mit einem Ziel einer Immuntherapie kodiert oder einen löslichen Rezeptor, ein Agonisten- oder Antagonisten-Peptid eines Rezeptors oder eines Adhäsionsproteins, ein künstliches, chimäres oder verkürztes Protein kodiert.

72. Kombinationsprodukt nach Anspruch 71, **dadurch gekennzeichnet, dass** die Nukleinsaure einen Anti-Idiotyp-Antikörper, ein lösliches Fragment des CD4-Rezeptors oder des TNFa-Rezeptors oder des Acetylcholinrezeptors kodiert.

73. Kombinationsprodukt nach einem der Ansprüche 69 bis 72, **dadurch gekennzeichnet, dass** die Nukleinsäure eine Vorstufe eines therapeutischen Proteins kodiert.

74. Kombinationsprodukt nach einem der Ansprüche 45 bis 73, **dadurch gekennzeichnet, dass** die Nukleinsäure in Form eines Plasmids vorliegt.

75. Kombinationsprodukt nach einem der Ansprüche 45 bis 73, **dadurch gekennzeichnet, dass** die Nukleinsäure ein Gen von grosser Grösse und/oder Introns und/oder regulatorische Elemente von kleiner oder von grosser Grösse enthalt.

76. Kombinationsprodukt nach einem der Ansprüche 45 bis 73, **dadurch gekennzeichnet, dass** die Nukleinsäure eine episomale DNA oder ein künstliches Hefe-Chromosom oder ein Minichromosom ist.

77. Kombinationsprodukt nach einem der Ansprüche 45 bis 76, **dadurch gekennzeichnet, dass** die Nukleinsäure Sequenzen enthält, welche die Expression des Transgens in dem Muskel erlauben und/oder begünstigen.

78. Kombinationsprodukt nach einem der Ansprüche 45 bis 77, **dadurch gekennzeichnet, dass** die Säure mit einer jeglichen Art von Vektoren oder einer jeglichen Kombination von Vektoren, welche erlauben, den Transfer von Nukleinsäure zu verbessern, wie Viren, synthetischen oder biosynthetischen Agentien oder ferner angetriebenen oder nicht-angetriebenen Kügelchen assoziiert ist.

79. Kombinationsprodukt nach einem der Ansprüche 45 bis 78, **dadurch gekennzeichnet, dass** der Muskel einer Behandlung, welche darauf abzielt, den Gentransfer zu verbessern, einer Behandlung von pharmakologischer Natur mit lokaler oder systemischer Anwendung oder einer enzymatischen, permeabilisierenden, chirurgischen, mechanischen, thermischen oder physikalischen Behandlung unterzogen wird.

80. Kombinationsprodukt nach einem der Ansprüche 45 bis 79, **dadurch gekennzeichnet, dass** es erlaubt, durch den Muskel ein Mittel in physiologischen und/oder therapeutischen Dosen, entweder in den Muskelzellen oder welches sekretiert wird, produzieren zu lassen.

81. Kombinationsprodukt nach einem der Ansprüche 45 bis 79, **dadurch gekennzeichnet, dass** es erlaubt, die Menge von exprimiertem Transgen zu modulieren, indem das Volumen von transfiziertem Muskelgewebe moduliert wird.

82. Kombinationsprodukt nach Anspruch 81, **dadurch gekennzeichnet, dass** es erlaubt, das Volumen von transfiziertem Muskelgewebe durch die Verwendung einer Mehrzahl von Verabreichungsstellen zu modulieren.

83. Kombinationsprodukt nach einem der Ansprüche 45 bis 82, **dadurch gekennzeichnet, dass** es erlaubt, die Menge von exprimiertem Transgen zu modulieren, indem die Anzahl, die Form, die Oberfläche und die Anordnung der Elektroden moduliert werden und indem die Intensität des Felds, die Anzahl, die Dauer, die Frequenz und die Form der Impulse sowie die Verabreichungsmenge und das Verabreichungsvolumen der Nukleinsäure variiert werden.

84. Kombinationsprodukt nach einem der Ansprüche 45 bis 83, **dadurch gekennzeichnet, dass** es erlaubt, die Lage der transfizierten Gewebe durch das Volumen von Gewebe, welches den örtlichen elektrischen Impulsen unterworfen wird, zu kontrollieren.

85. Kombinationsprodukt nach einem der Ansprüche 45 bis 84, **dadurch gekennzeichnet, dass** es eine Rückkehr zu der Ausgangssituation durch operative Entfernung der Zone von transfiziertem Gewebe erlaubt.
